(19) Europäisches Patentamt
European Patent Office
Office européen des brevets

(11) EP 2 155 745 B1

(12) **EUROPÄISCHE PATENTSCHRIFT**

(45) Veröffentlichungstag und Bekanntmachung des Hinweises auf die Patenterteilung:
**28.01.2015 Patentblatt 2015/05**

(21) Anmeldenummer: **08758743.2**

(22) Anmeldetag: **23.05.2008**

(51) Int Cl.:
***C07D 471/04*** *(2006.01)* ***A61K 31/437*** *(2006.01)*
***A61K 31/506*** *(2006.01)* ***A61P 25/00*** *(2006.01)*
***A61P 31/18*** *(2006.01)*

(86) Internationale Anmeldenummer:
**PCT/EP2008/004151**

(87) Internationale Veröffentlichungsnummer:
**WO 2008/155000 (24.12.2008 Gazette 2008/52)**

(54) **6-(Pyrrolopyridinyl)- Pyrimidin-2-yl-Amin-Derivate und ihre Verwendung zur Behandlung von Krebs und Aids**

6-(pyrrolopyridinyl)-pyrimidine-2-yl-amine derivatives and their use for the treatment of cancers and aids

Dérivés de 6-(pyrrolopyridinyl)- pyrimidine-2-yl-amine et leur utilisation pour le traitement des cancers et le sida

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **21.06.2007 DE 102007028515**

(43) Veröffentlichungstag der Anmeldung:
**24.02.2010 Patentblatt 2010/08**

(73) Patentinhaber: **Merck Patent GmbH**
**64293 Darmstadt (DE)**

(72) Erfinder:
- **DORSCH, Dieter**
  **64372 Ober-Ramstadt (DE)**
- **WUCHRER, Margarita**
  **64291 Darmstadt (DE)**
- **BURGDORF, Lars, Thore**
  **60389 Frankfurt am Main (DE)**
- **SIRRENBERG, Christian**
  **64289 Darmstadt (DE)**
- **ESDAR, Christina**
  **55128 Mainz (DE)**
- **MUELLER, Thomas, J., J.**
  **40591 Duesseldorf (DE)**
- **MERKUL, Eugen**
  **40591 Duesseldorf (DE)**

(56) Entgegenhaltungen:
**WO-A-2005/095400 WO-A-2006/124863**
**WO-A-2007/107221**

- **FRESNEDA P M ET AL: "Synthesis of the indole alkaloids meridianins from the tunicate Aplidium meridianum" TETRAHEDRON, ELSEVIER SCIENCE PUBLISHERS, AMSTERDAM, NL, Bd. 57, Nr. 12, 17. März 2001 (2001-03-17), Seiten 2355-2363, XP004231517 ISSN: 0040-4020 in der Anmeldung erwähnt**
- **HUANG ET AL: "Synthesis of 2-amino-4-(7-azaindol-3-yl)pyrimidines as cyclin dependent kinase 1 (CDK1) inhibitors" BIOORGANIC & MEDICINAL CHEMISTRY LETTERS, PERGAMON, ELSEVIER SCIENCE, GB, Bd. 16, Nr. 18, 15. September 2006 (2006-09-15), Seiten 4818-4821, XP005594968 ISSN: 0960-894X**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft Verbindungen der Formel I

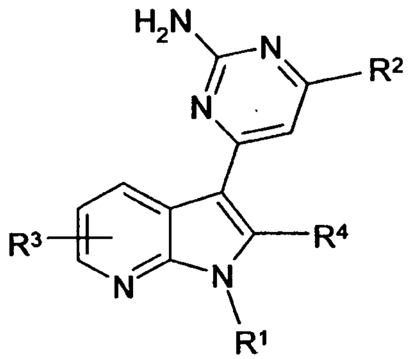

I

worin

| | |
|---|---|
| $R^1$ | H, A oder $-[C(R^5)_2]_nAr$, |
| $R^2$ | A, $-[C(R^5)_2]_nAr$, $-[C(R^5)_2]_nHet$, $-[C(R^5)_2]_n$Cycloalkyl, $-[C(R^5)_2]_nOR^5$, $-[C(R^5)_2]_nN(R^5)_2$ oder $-[C(R^5)_2]_nCOOR^5$ |
| $R^3$ | H, A, Hal, CN, $-[C(R^5)_2]_nAr$, $-[C(R^5)_2]_nHet$ oder $-[C(R^5)_2]_n$Cycloalkyl oder $-[C(R^5)_2]_nN(R^5)_2$, |
| $R^4$ | H, A, $-[C(R^5)_2]_nAr$, $-[C(R^5)_2]_nHet$ oder $-[C(R^5)_2]_n$Cycloalkyl, |
| $R^5$ | H oder Alkyl mit 1-6 C-Atomen, |
| A | unverzweigtes oder verzweigtes Alkyl mit 1-10 C-Atomen, worin eine oder zwei $CH_2$-Gruppen durch O- oder S-Atome und/oder durch -CH=CH-Gruppen und/oder auch 1-7 H-Atome durch F ersetzt sein können, |
| Cycloalkyl | Cyclisches Alkyl mit 3-7 C-Atomen, das zusätzlich durch Alkyl mit 1-6 C-Atomen substituiert sein kann, |
| Hal | F, Cl, Br oder I, |
| Ar | unsubstituiertes oder ein-, zwei- oder dreifach durch Hal, A, $OR^5$, $N(R^5)_2$, $NO_2$, CN, $COOR^5$, $CON(R^5)_2$, $NR^5COA$, $NR^5SO_2A$, $COR^5$, $SO_2N(R^5)_2$ und/oder $S(O)_pA$ substituiertes Phenyl, |
| Het | einen unsubstituierten oder ein-, zwei- oder dreifach durch Hal, A, $OR^5$, $N(R^5)_2$, $NO_2$, CN, $COOR^5$, $CON(R^5)_2$, $NR^5COA$, $NR^5SO_2A$, $COR^5$, $SO_2NR^5$, $S(O)_pA$ und/oder =0 (Carbonylsauerstoff) substituierten ein- oder zweikernigen gesättigten, ungesättigten oder aromatischen Heterocyclus mit 1 bis 4 N-, und/oder O- und/oder S-Atomen, |
| n | 0, 1, 2, 3 oder 4, |
| p | 0, 1 oder 2 |

bedeuten,

sowie ihre pharmazeutisch verwendbaren Solvate, Salze, Tautomere und Stereoisomere, einschließlich deren Mischungen in allen Verhältnissen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze und/oder Solvate bei guter Verträglichkeit sehr wertvolle pharmakologische Eigenschaften besitzen.

Insbesondere zeigen sie eine inhibierende Wirkung der Zellproliferation/ Zellvitalität als Antagonisten oder Agonisten. Die erfindungsgemäßen Verbindungen können daher zur Bekämpfung und/oder Behandlung von Tumoren, Tumorwachstum und/oder Tumormetastasen verwendet werden.

Die antiproliferative Wirkung kann in einem Proliferationsassay/ Vitalitätsassay getestet werden.

Andere 4-(Pyrrolopyridinyl)-pyrimidinyl-2-amin-derivate sind beispielsweise von P.M. Fresneda et al. in Tetrahedron 57 (2001) 2355-2363 beschrieben.

Andere 4-(Pyrrolopyridinyl)-pyrimidinyl-2-amin-derivate beschreibt auch A. Karpov in seiner Dissertation, Universität Heidelberg, April 2005.

Andere Amino-pyridinderivate, die einen 2,2,6,6-Tetramethyl-piperidin-4-yl-Rest tragen, sind zur Behandlung von entzündlichen und Autoimmunerkrankungen in WO 2004/089913 beschrieben.

Andere 6-(Pyrrolopyridinyl)pyrimidin-2-yl-amin-derivate und ihre Verwendung zur Behandlung von Krebserkrankungen sind in WO 2005/095400 A, WO 2006/124863 A und in Bioorganic & Medicinal Chemistry Letters, Vol. 16, Nr. 18, 4818-4821, 2006, XP005594968 beschrieben.

Dementsprechend werden die erfindungsgemäßen Verbindungen oder ein pharmazeutisch unbedenkliches Salz davon für die Behandlung von Krebs verabreicht, einschließlich solider Karzinome, wie zum Beispiel Karzinome (z. B. der Lungen, des Pankreas, der Schilddrüse, der Harnblase oder des Kolons), myeloische Erkrankungen (z. B. myeloische Leukämie) oder Adenome (z. B. villöses Kolonadenom).
Zu den Tumoren zählen weiterhin die Monozytenleukämie, Hirn-, Urogenital-, Lymphsystem-, Magen-, Kehlkopf- und Lungenkarzinom, darunter Lungenadenokarzinom und kleinzelliges Lungenkarzinom, Bauchspeicheldrüsen- und/oder Brustkarzinom.

Die Verbindungen sind ferner nützlich bei der Behandlung der durch HIV-1 (Human Immunodeficiency Virus Typ 1) induzierten Immunschwäche.

Als krebsartige hyperproliferative Erkrankungen sind Hirnkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischer Krebs, Schilddrüsenkrebs, Lymphome, chronische Leukämie und akute Leukämie anzusehen. Insbesondere krebsartiges Zellwachstum ist eine Erkrankung, die ein Ziel der vorliegenden Erfindung darstellt. Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Es kann gezeigt werden, dass die erfindungsgemäßen Verbindungen antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einher-gehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vörbestehender Leiden verwendet. Die Verhinderung von Proliferation/ Vitalität wird durch Verabreichung der erfindungsgemäßen Verbindungen vor Entwicklung der evidenten Krankheit erreicht, z. B. zur Verhinderung des Tumorwachstums. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer inkubiert, die ausreicht, um den aktiven Mitteln zu ermöglichen, Zelltod zu induzieren oder Zellproliferation, Zellvitalität oder Migration zu inhibieren, gewöhnlich zwischen ungefähr einer Stunde und einer Woche. Zum Testen in vitro können kultivierte Zellen aus einer Biopsieprobe verwendet werden. Die Menge nach der Behandlung zurückbleibenden Zellen werden dann bestimmt.
Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Es gibt viele mit einer Deregulation der Zellproliferation und des Zelltods (Apoptose) einhergehende Erkrankungen. Die Leiden von Interesse schließen die folgenden Leiden ein, sind aber nicht darauf beschränkt. Die erfindungsgemäßen Verbindungen sind nützlich bei der Behandlung einer Reihe verschiedener Leiden, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

Die Verbindungen der Formel I, wirken auch als Regulatoren, Modulatoren oder Inhibitoren von Proteinkinasen, insbesondere des Typs Serin/Threonin-Kinase, zu denen unter anderem die Phosphoinositidabhängige Kinase (PDK) gehören. Eine gewisse Wirkung zeigen die erfindungsgemäßen Verbindungen bei der Inhibierung der Serin/Threonin-Kinase PDK1.

PDK1 phosphoryliert und aktiviert eine Untergruppe der AGC Proteinkinasen-Familie, umfassend PKB, SGK,

S6K und PKC Isoformen. Diese Kinasen sind an dem PI3K Signalübertragungsweg beteiligt und kontrollieren grundlegende zelluläre Funktionen wie Überleben, Wachstum und Differenzierung. PDK1 ist somit ein bedeutender Regulator diverser metabolischer, proliferativer und Lebenserhaltungs-Effekte.

Durch Proteinkinasen hervorgerufene Erkrankungen sind durch eine anomale Aktivität oder Hyperaktivität solcher Proteinkinasen gekennzeichnet. Anomale Aktivität betrifft entweder: (1) die Expression in Zellen, die gewöhnlich diese Proteinkinasen nicht exprimieren; (2) erhöhte Kinasen-Expression, die zu unerwünschter Zellproliferation, wie Krebs, führt; (3) erhöhte Kinasen-Aktivität, die zu unerwünschter Zellproliferation, wie Krebs, und/oder zu Hyperaktivität der entsprechenden Proteinkinasen führt. Hyperaktivität bezieht sich entweder auf eine Amplifikation des Gens, das eine bestimmte Proteinkinase codiert, oder die Erzeugung eines Aktivitäts-Spiegels, der mit einer Zellproliferationserkrankung korreliert werden kann (d.h. mit steigendem Kinase-Spiegel steigt die Schwere eines oder mehrerer Symptome der Zellproliferationserkrankung) die biologische Verfügbarkeit einer Proteinkinase kann auch durch das Vorhandensein oder Fehlen eines Satzes von Bindungsproteinen dieser Kinase beeinflusst werden.

Die wichtigsten Krebsarten, die unter Verwendung einer erfindungsgemäßen Verbindung behandelt werden können, umfassen Kolorektalkrebs, kleinzelligen Lungenkrebs, nicht-kleinzelligen Lungenkrebs, das multiple Myelom sowie das Nierenzellkarzinom und das Endometriumkarzinom, bersonders auch Krebsarten, in denen PTEN mutiert ist, u. a. Brustkrebs, Prostata-Krebs und Glioblastom.

Zudem können die erfindungsgemäßen Verbindungen verwendet werden, um bei gewissen existierenden Krebs-Chemotherapien und -bestrahlungen additive oder synergistische Effekte zu erzielen und/oder, um die Wirksamkeit gewisser existierender Krebs-Chemotherapien und -bestrahlungen wiederherzustellen.

Gegenstand der Erfindung sind auch die optisch aktiven Formen (Stereoisomeren), Salze, die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.

Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.

Hierzu gehören auch bioabbaubare Polymerderivate der erfindungsgemäßen Verbindungen, wie dies z. B. in Int. J. Pharm. 115, 61-67 (1995) beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:

verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder
einer Störung.

Die Bezeichnung "therapeutisch wirksame Menge" umfaßt auch die Mengen, die wirkungsvoll sind, die normale physiologische Funktion zu erhöhen.

Gegenstand der Erfindung ist auch die Verwendung von Mischungen der Verbindungen der Formel I, z.B. Gemische zweier Diastereomerer z.B. im Verhältnis 1:1, 1:2, 1:3, 1:4, 1:5, 1:10, 1:100 oder 1:1000.

Besonders bevorzugt handelt es sich dabei um Mischungen stereoisomerer Verbindungen.

Gegenstand der Erfindung sind die Verbindungen der Formel I und ihre Salze sowie ein Verfahren zur Herstellung von Verbindungen der Formel I nach den Ansprüchen 1-13 sowie ihrer pharmazeutisch verwendbaren Salze, Solvate, Tautomeren und Stereoisomeren, dadurch gekennzeichnet, daß man

a) eine Verbindung der Formel II

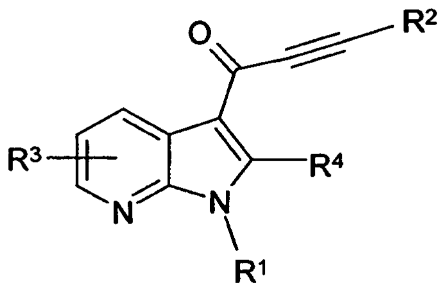

II

,

worin $R^1$ eine Indolschutzgruppe bedeutet,
$R^2$, $R^3$ und $R^4$ die in Anspruch 1 angegebenen Bedeutungen haben,
mit Guanidin umsetzt,
und gleichzeitig oder anschließend die Indolschutzgruppe abspaltet,
oder

b) in einer Verbindung der Formel I einen Rest $R^2$ in einen anderen $R^2$ umwandelt,
indem man

i) eine Aminoschutzgruppe abspaltet,
ii) ein Imid zu einem Amin spaltet,
iii) eine Alkylierung durchführt,

und/oder eine Base oder Säure der Formel I in eines ihrer Salze umwandelt.

Vor- und nachstehend haben die Reste $R^1$, $R^2$, $R^3$ und $R^4$ die bei der Formel I angegebenen Bedeutungen, sofern nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.
In A können auch eine oder zwei $CH_2$-Gruppen durch O- oder S-Atome und/oder durch -CH=CH-Gruppen ersetzt sein.
So bedeutet A z.B. auch 2-Methoxy-ethyl.

Cycloalkyl bedeutet vorzugsweise Cyclopropyl, Cyclobutyl, Cylopentyl, Cyclohexyl oder Cycloheptyl.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Trifluormethylphenyl, o-, m- oder p-Fluorphenyl, o-, m- oder p-Bromphenyl, o-, m- oder p-Chlorphenyl, o-, m- oder p-Hydroxyphenyl, o-, m- oder p-Methoxyphenyl, o-, m- oder p-Methylsulfonylphenyl, o-, m- oder p-Nitrophenyl, o-, m- oder p-Aminophenyl, o-, m- oder p-Methylaminophenyl, o-, m- oder p-Dimethylaminophenyl, o-, m- oder p-Aminosulfonylphenyl, o-, m- oder p-Methylaminosulfonylphenyl, o-, m- oder p-Aminocarbonylphenyl, o-, m- oder p-Carboxyphenyl, o-, m- oder p-Methoxycarbonylphenyl, o-, m- oder p-Ethoxycarbonylphenyl, o-, m- oder p-Acetylphenyl, o-, m- oder p-Formylphenyl, o-, m- oder p-Cyanphenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3,4-, 2,3,5-, 2,3,6-, 2,4,6- oder 3,4,5-Trichlorphenyl, p-lodphenyl, 4-Fluor-3-chlorphenyl, 2-Fluor-4-bromphenyl, 2,5-Difluor-4-bromphenyl oder 2,5-Dimethyl-4-chlorphenyl.

Ar bedeutet vorzugsweise unsubstituiertes oder ein-, zwei- oder dreifach durch A, $NO_2$, $N(R^5)_2$, CN und/oder $COOR^5$ substituiertes Phenyl.

Het bedeutet, ungeachtetet weiterer Substitutionen, z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 2-, 3-, 4-, 5-, 6- oder 7-Indazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benz-oxadiazol-5-yl.
Die heterocyclischen Reste können auch teilweise oder vollständig hydriert sein.

Unsubstituiertes Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-,-3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-,-2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-, -2-, -3-, -4-, -5-, -6-, -7- oder -8-isochinolyl, 2-, 3-, 5-, 6-, 7- oder 8- 3,4-Dihydro-2H-benzo[1,4]-oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxomethylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Het bedeutet weiterhin vorzugsweise einen unsubstituierten oder ein- oder zwei- oder dreifach durch A, OH, OA, Hal, $CON(R^5)_2$, $N(R^5)_2$ und/oder =O (Carbonylsauerstoff) substituierten ein- oder zweikernigen gesättigten, ungesättigten oder aromatischen Heterocyclus mit 1 bis 3 N- und/oder O-Atomen;
weiterhin besonders bevorzugt unsubstituiertes oder ein-, zwei oder dreifach durch A, OH, OA, Hal, $CON(R^5)_2$, $N(R^5)_2$ und/oder =O substituiertes Dihydro-pyrrolyl, Pyrrolidinyl, Tetrahydro-imidazolyl, Dihydropyrazolyl, Tetrahydro-pyrazolyl, Dihydro-pyridyl, Tetrahydro-pyridyl, Piperidinyl, Morpholinyl, Hexahydro-pyridazinyl, Hexahydro-pyrimidinyl, Piperazinyl, 1,2,3,4-Tetrahydro-chinolyl, 1,2,3,4-Tetrahydro-isochinolyl, 3,4-Dihydro-benzo[1,4]oxazinyl, 2,3-Dihydro-isoindolyl, 2,3-Dihydro-indolyl, Oxazolidinyl, Isoxazolidinyl, Oxadiazolidinyl, Thiazolidinyl, Furyl, Thienyl, Pyrrolyl, Imidazolyl, Pyrazolyl, Oxazolyl, Isoxazolyl, Thiazolyl, Pyridyl, Pyrimidinyl, Triazolyl, Tetrazolyl, Thiadiazol, Pyridazinyl, Pyrazinyl, Indolyl, Isoindolyl, Benzimidazolyl, Indazolyl, Chinolyl oder 1,3-Benzodioxolyl.

Het bedeutet ganz besonders bevorzugt unsubstituiertes oder ein- oder zwei oder dreifach durch A, $CON(R^5)_2$, $N(R^5)_2$ oder F und/oder =O substituiertes Dihydro-pyrrolyl, Pyrrolidinyl, Tetrahydro-imidazolyl, Dihydro-pyrazolyl, Tetrahydro-pyrazolyl, Dihydro-pyridyl, Tetrahydro-pyridyl, Piperidinyl, Morpholinyl, Hexahydro-pyridazinyl, Hexahydro-pyrimidinyl, Piperazinyl, 1,2,3,4-Tetrahydro-chinolyl, 1,2,3,4-Tetrahydro-isochinolyl, 3,4-Dihydro-benzo[1,4]oxazinyl, 2,3-Dihydro-isoindolyl oder 2,3-Dihydro-indolyl, Pyrazolyl, Pyridyl, Furyl oder Isooxazol.

$R^1$ bedeutet vorzugsweise H oder $-[C(R^5)_2]_nAr$.
$R^2$ bedeutet A, $-[C(R^5)_2]_nAr$, $-[C(R^5)_2]_nHet$, $-[C(R^5)_2]_nCycloalkyl$,
$-[C(R^5)_2]_nOR^5$, $-[C(R^5)_2]_nN(R^5)2$ oder $-[C(R^5)_2]_nCOOR^5$
vorzugsweise Methyl, Ethyl, Propyl, Butyl, Benzyl, Phenyl, o-, m- oder p-Tolyl, o-, m- oder p-Ethylphenyl, o-, m- oder p-Propylphenyl, o-, m- oder p-Isopropylphenyl, o-, m- oder p-Butylphenyl, o-, m- oder p-tert.-Butylphenyl, o-, m- oder p-Trifluormethylphenyl, 4-Propylcyclohexyl, Methoxymethyl, Hydroxymethyl, 1,3-Dioxo-isoindol-2-yl-methyl, 1,3-Dioxo-isoindol-2-yl-ethyl, 1,3-Dioxo-isoindol-2-yl-propyl, 1,3-Dioxo-isoindol-2-yl-butyl, Aminomethyl, Aminoethyl, Aminopropyl, Aminobutyl, Dimethylaminomethyl, Dimethylaminopropyl, Pyrrolidin-1-yl-propyl, Piperidin-4-yl-methyl, 1-Methyl-piperidn-4-yl-methyl, Piperidin-4-yl-propyl, 1-Methyl-piperidin-4-yl-methyl, Pyrrolidin-2-yl-ethyl oder 3-Morpholin-4-yl-propyl, 3-Piperazin-1-yl-propyl, Aminophenyl, 3-Carboxy-propyl, Nitrophenyl, 4-Hydroxy-butyl, 3-Hydroxy-propyl, Hydroxy-methyl, 2-Carboxy-ethyl oder 3-(4-Methyl-piperazin-1-yl)-propyl.

$R^3$ bedeutet vorzugsweise H, Br, Cl, CN, Ar oder Het
$R^4$ bedeutet vorzugsweise H oder A
$R^5$ bedeutet vorzugsweise H oder A

Hal bedeutet vorzugsweise F, Cl oder Br, aber auch I, besonders bevorzugt F oder Cl.

Für die gesamte Erfindung gilt, daß sämtliche Reste, die mehrfach auftreten, gleich oder verschieden sein können, d.h. unabhängig voneinander sind.
Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Il ausgedrückt werden, die der Formel I entsprechen und worin die nicht näher bezeichneten Reste die bei der Formel I angegebene Bedeutung haben, worin jedoch

in Ia $R^1$ H oder $-[C(R^5)_2]_nAr$

bedeutet;

in Ib $R^2$ Methyl, Ethyl, Propyl, Butyl, Benzyl, Phenyl, o-, m- oder p-Tolyl, o-, m- oder p-Ethylphenyl, o-, m- oder p-Propylphenyl, o-, m- oder p-Isopropylphenyl, o-, m- oder p-Butylphenyl, o-, m- oder p-tert.-Butylphenyl, o-, m- oder p-Trifluormethylphenyl, 4-Propylcyclohexyl, Methoxymethyl, Hydroxymethyl, 1,3-Dioxo-isoindol-2-yl-methyl, 1,3-Dioxo-isoindol-2-yl-ethyl, 1,3-Dioxo-isoindol-2-yl-propyl, 1,3-Dioxo-isoindol-2-yl-butyl, Aminomethyl, Aminoethyl, Aminopropyl, Aminobutyl, Dimethylaminomethyl, Dimethylaminopropyl, Pyrrolidin-1-yl-propyl, Piperidin-4-yl-methyl, 1-Methyl-piperidn-4-yl-methyl, Piperidin-4-yl-propyl, 1-Methyl-piperidin-4-yl-methyl, Pyrrolidin-2-yl-ethyl oder 3-Morpholin-4-yl-propyl, 3-Piperazin-1-yl-propyl, Aminophenyl, 3-Carboxy-propyl, Nitrophenyl, 4-Hydroxy-butyl, 3-Hydroxy-propyl, Hydroxy-methyl, 2-Carboxy-ethyl oder 3-(4-Methyl-piperazin-1-yl)-propyl.

bedeutet;

in Ic $R^3$ H, Br, Cl, CN, Ar oder Het bedeutet;

in Id $R^4$ H oder A bedeutet;

in Ie $R^5$ H oder A bedeutet;

in If A unverzweigtes oder verzweigtes Alkyl mit 1-8 C-Atomen, worin eine oder zwei $CH_2$-Gruppe durch Sauerstoff und/oder auch 1-7 H-Atome durch F ersetzt sein können,

bedeutet;

in Ig A unverzweigtes oder verzweigtes Alkyl mit 1-6 C-Atomen, worin eine oder zwei $CH_2$-Gruppe durch Sauerstoff und/oder 1-7 H-Atome durch F ersetzt sein können,

bedeutet;

in Ih Ar unsubstituiertes oder ein-, zwei- oder dreifach durch A, $NO_2$, $N(R^5)_{2,}$ CN und/oder $COOR^5$ substituiertes Phenyl,

bedeutet;

in Ii Het einen unsubstituierten oder ein- oder zwei- oder dreifach durch A, OH, OA, Hal, $CON(R^5)_2$, $N(R^5)_2$ und/oder =O (Carbonylsauerstoff) substituierten ein- oder zweikernigen gesättigten, ungesättigten oder aromatischen Heterocyclus mit 1 bis 3 N- und/oder O-Atomen,

bedeutet;

in Ij Het unsubstituiertes oder ein- oder zwei- oder dreifach durch A, OH, OA, Hal, $CON(R^5)_2$, $N(R^5)_2$ und/oder =O substituiertes Dihydro-pyrrolyl, Pyrrolidinyl, Tetrahydroimidazolyl, Dihydro-pyrazolyl, Tetrahydro-pyrazolyl, Dihydro-pyridyl, Tetrahydro-pyridyl, Piperidinyl, Morpholinyl, Hexahydro-pyridazinyl, Hexahydropyrimidinyl, Piperazinyl, 1,2,3,4-Tetrahydro-chinolyl, 1,2,3,4-Tetrahydro-isochinolyl, 3,4-Dihydro-benzo[1,4]oxazinyl, 2,3-Dihydro-isoindolyl, 2,3-Dihydro-indolyl, Oxazolidinyl, Isoxazolidinyl, Oxadiazolidinyl, Thiazolidinyl, Furyl, Thienyl, Pyrrolyl, Imidazolyl, Pyrazolyl, Oxazolyl, Isoxazolyl, Thiazolyl, Pyridyl, Pyrimidinyl, Triazolyl, Tetrazolyl, Thiadiazol, Pyridazinyl, Pyrazinyl, Indolyl, Isoindolyl, Benzimidazolyl, Indazolyl, Chinolyl oder 1,3-Benzodioxolyl, bedeutet;

in Ik Het unsubstituiertes oder ein- oder zwei-, oder dreifach durch A, $CON(R^5)_2$, $N(R^5)_2$ oder F und/oder =O substituiertes Dihydro-pyrrolyl, Pyrrolidinyl, Tetrahydroimidazolyl, Dihydro-pyrazolyl, Tetrahydro-pyrazolyl, Dihydro-pyridyl, Tetrahydro-pyridyl, Piperidinyl, Morpholinyl, Hexahydro-pyridazinyl, Hexahydropyrimidinyl, Piperazinyl, 1,2,3,4-Tetrahydro-chinolyl, 1,2,3,4-Tetrahydro-isochinolyl, 3,4-Dihydro-benzo[1,4]oxazinyl, 2,3-Dihydro-isoindolyl oder 2,3-Dihydro-indolyl, Pyrazolyl, Pyridyl, Furyl oder Isooxazol

bedeutet;

in Il $R^1$ H oder $-[C(R^5)_2]_nAr$,

$R^2$ A, $-[C(R^5)_2]_nAr$, $-(C(R^5)_2]_nHet$, $-[C(R^5)_2]_nCycloalkyl$, $-[C(R^5)_2]_nOR^5$, $-(C(R^5)_2]_nN(R^5)_2$ oder $-(C(R^5)_2]_nCOOR^5$

$-[C(R^5)_2]_nCOOR^5$

(fortgesetzt)

| | |
|---|---|
| $R^3$ | H, Br, Cl, CN, Ar oder Het |
| $R^4$ | H oder A, |
| $R^5$ | H oder A, |
| A | unverzweigtes oder verzweigtes Alkyl mit 1-6 C-Atomen, worin eine oder zwei $CH_2$-Gruppen durch Sauerstoff und/oder 1-7 H-Atome durch F ersetzt sein können, |
| Ar | unsubstituiertes oder ein-, zwei- oder dreifach durch A, $NO_2$, $N(R^5)_2$, CN und/oder $COOR^5$ substituiertes Phenyl, |
| Het | unsubstituiertes oder ein- oder zwei- oder dreifach durch A, $CON(R^5)_2$, $N(R^5)_2$ oder F und/oder =O substituiertes Dihydro-pyrrolyl, Pyrrolidinyl, Tetrahydro-imidazolyl, Dihydro-pyrazolyl, Tetrahydro-pyrazolyl, Dihydro-pyridyl, Tetrahydro-pyridyl, Piperidinyl, Morpholinyl, Hexahydro-pyridazinyl, Hexahydro-pyrimidinyl, Piperazinyl, 1,2,3,4-Tetrahydro-chinolyl, 1,2,3,4-Tetrahydro-isochinolyl, 3,4-Dihydro-benzo[1,4]oxazinyl, 2,3-Dihydro-isoindolyl oder 2,3-Dihydro-indolyl, Pyrazolyl, Pyridyl, Furyl oder Isooxazol |
| n | 0, 1, 2, 3 oder 4, |

bedeuten;
sowie ihre pharmazeutisch verwendbaren Salze, Solvate, Tautomere und Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Verbindungen der Formel I können vorzugsweise erhalten werden, indem man Verbindungen der Formel II und mit einem Guanidinsalz, wie z.B. Guanidiniumcarbonat, umsetzt.
Die Verbindungen der Formel II sind in der Regel bekannt. Sind sie neu, so können sie aber nach an sich bekannten Methoden hergestellt werden.

Die Umsetzung erfolgt in einem inerten Lösungsmittel und erfolgt in der Regel in Gegenwart eines säurebindenden Mittels vorzugsweise einer organischen Base wie DIPEA, Triethylamin, Dimethylanilin, Pyridin oder Chinolin.
Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Die Reaktionszeit liegt je nach den angewendeten Bedingungen zwischen einigen Minuten und 14 Tagen, die Reaktionstemperatur zwischen etwa -15° und 150°, normalerweise zwischen 40° und 130°, besonders bevorzugt zwischen 60° und 110°C.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylen-glykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt sind Glykolether, wie Ethylenglycolmonomethylether, THF, Dichlormethan und/oder DMF.

Bevorzugte Indolschutzgruppen sind z.B. Sulfonylschutzgruppen, wie Tosyl oder Mesyl, ferner Schutzgruppen wie z.B. BOC.

Die Spaltung eines Ethers erfolgt unter Methoden, wie sie dem Fachmann bekannt sind.
Eine Standardmethode zur Etherspaltung, z.B. eines Methylethers, ist die Verwendung von Bortribromid.
Hydrogenolytisch entfernbare Gruppen, z.B. die Spaltung eines Benzylethers, können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt.

Ester können z.B. mit Essigsäure oder mit NaOH oder KOH in Wasser, Wasser-THF oder Wasser-Dioxan bei Temperaturen zwischen 0 und 100° verseift werden.

Alkylierungen am Stickstoff erfolgen unter Standardbedingungen, wie sie dem Fachmann bekannt sind.

Die Verbindungen der Formeln I können ferner erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, z. B. solche, die der Formel I entsprechen, aber anstelle einer $NH_2$-Gruppe eine NHR'-Gruppe (worin R' eine Aminoschutzgruppe bedeutet, z. B. BOC oder CBZ) enthalten.

Ferner sind Ausgangsstoffe bevorzugt, die anstelle des H-Atoms einer Hydroxygruppe eine Hydroxyschutzgruppe tragen, z. B. solche, die der Formel I entsprechen, aber anstelle einer Hydroxyphenylgruppe eine R"O-phenylgruppe enthalten (worin R" eine Hydroxyschutzgruppe bedeutet).

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-Iodethoxycarbonyl; Aralkyloxycarbonyl wie CBZ ("Carbobenzoxy"), 4-Methoxybenzyloxycarbonyl, FMOC; Arylsulfonyl wie Mtr, Pbf oder Pmc. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butoxycarbonyl, Benzyl, p-Nitrobenzoyl, p-Toluolsulfonyl, tert.-Butyl und Acetyl, wobei Benzyl und tert.-Butyl besonders bevorzugt sind. Die COOH-Gruppen in Asparaginsäure und Glutaminsäure werden bevorzugt in Form ihrer tert.-Butylester geschützt (z. B. Asp(OBut)).

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut, Pbf, Pmc und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie ($C_1$-$C_4$) Alkylhalogeniden, z.B. Methyl-, Ethyl-, Isopropyl- und tert.-Butylchlorid, -bromid und -iodid; Di($C_1$-$C_4$)Alkylsulfaten, z.B. Dimethyl-, Diethyl- und Diamylsulfat; ($C_{10}$-$C_{18}$)Alkylhalogeniden, z.B. Decyl-, Dodecyl-, Lauryl-, Myristyl- und Stearylchlorid, -bromid und -iodid; sowie Aryl-($C_1$-$C_4$)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin,

Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel I und/oder ihre pharmazeutisch verwendbaren Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I sowie Salze und Solvate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie die Salze und Solvate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinyl-pyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels Iontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein

Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine Verbindung der Formel I und/oder ihre pharmazeutisch verwendbaren Salze, Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von

(a) einer wirksamen Menge an einer Verbindung der Formel I und/oder ihrer pharmazeutisch verwendbaren Salze, Solvate und Stereoisomere, einschließlich deren Mischungen in allen Verhältnissen, und

(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Salze, Solvate und Stereoisomere, einschließlich deren Mischungen in allen Verhältnissen,

und einer wirksamen Menge eines weiteren Arzneimittelwirkstoffs gelöst oder in lyophilisierter Form vorliegt.

## VERWENDUNG

Die vorliegenden Verbindungen eignen sich als pharmazeutische Wirkstoffe für Säugetiere, insbesondere für den Menschen, bei der Behandlung und Bekämpfung von Krebserkrankungen.

Die vorliegende Erfindung umfasst die Verwendung der Verbindungen der Formel I und/oder ihre physiologisch unbedenklichen Salze und Solvate zur Herstellung eines Arzneimittels zur Behandlung oder Vorbeugung von Krebs. Bevorzugte Karzinome für die Behandlung stammen aus der Gruppe Hirnkarzinom, Urogenitaltraktkarzinom, Karzinom des lymphatischen Systems, Magenkarzinom, Kehlkopfkarzinom und Lungenkarzinom Darmkrebs. Eine weitere Gruppe bevorzugter Krebsformen sind Monozytenleukämie, Lungenadenokarzinom, kleinzellige Lungenkarzinome, Bauchspeicheldrüsenkrebs, Glioblastome und Brustkarzinom.

Ebenfalls umfasst ist die Verwendung der Verbindungen der Formel I und/oder ihre physiologisch unbedenklichen Salze und Solvate zur Herstellung eines Arzneimittels zur Behandlung und/oder Bekämpfung einer durch Tumore bedingten Krankheit bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.

Insbesondere bevorzugt ist die Verwendung zur Behandlung einer Krankheit, wobei die Krankheit ein fester Tumor ist.

Der feste Tumor ist vorzugsweise ausgewählt aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopf und/oder der Lunge.

Der feste Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt ist die Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Gegenstand der Erfindung ist weiterhin die Verwendung der erfindungsgemäßen Verbindungen zur Behandlung von Knochen-Pathologien, wobei die Knochenpathologie aus der Gruppe Osteosarkom, Osteoarthritis und Rachitis stammt.

Die Verbindungen der Formel I können auch gemeinsam mit anderen gut bekannten Therapeutika, die aufgrund ihrer jeweiligen Eignung für das behandelte Leiden ausgewählt werden, verabreicht werden.

Die vorliegenden Verbindungen eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-Protease-Hemmer, Reverse-Transkriptase-Hemmer sowie weitere Angiogenesehemmer. Die vorliegenden Verbindungen eignen sich insbesondere zur gemeinsamen Anwendung mit Radiotherapie. "Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1-piperidinyl)ethoxy]phenyl]-2H-1-benzopyran-3-yl]phenyl-2,2-dimethylpropanoat, 4,4'-Dihydroxybenzo-phenon-2,4-dinitrophenylhydrazon und SH646, was jedoch keine Einschränkung darstellen soll.

"Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5$\alpha$-Reduktase-Hemmer, Nilutamid, Flutamid, Bicalutamid, Liarozol und Abirateron-acetat.

"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, $\alpha$-Difluormethylornithin, ILX23-7553, trans-N-(4'-Hydroxyphenyl)retinamid und N-4-Carboxyphenylretinamid.

"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkaliernde Mittel, Mikrotubulin-Hemmer und Topoisomerase-Hemmer.

Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis-[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyl-daunorubicin (siehe WO 00/50032), was jedoch keine Einschränkung darstellen soll.

Zu den Mikrotubulin-Hemmern zählen zum Beispiel Paclitaxel, Vindesinsulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.

Topoisomerase-Hemmer sind zum Beispiel Topotecan, Hycaptamin, Irinotecan, Rubitecan, 6-Ethoxypropionyl-3,4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNPI1100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropylamino)-7,10-dihydroxy-2-(2-hydroxyethylaminomethyl)-6H-pyrazolo[4,5,1-de]-acridin-6-on, N-[1-[2(Diethylamino)ethylamino]-7-methoxy-9-oxo-9H-thioxanthen-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna.

Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabin-ocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-mannoheptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diazatetracyclo(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehyd-thiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

**Wirkungsnachweis von pharmakologischen Inhibitoren auf die Proliferation/Vitalität von Tumorzellen *in vitro***

**1.0 Hintergrund**

In der vorliegenden Versuchsbeschreibung wird die Hemmung der Tumorzellproliferation/ Tumorzellvitalität durch Wirkstoffe beschrieben. Die Zellen werden in geeigneter Zelldichte in Mikrotiterplatten (96-well Format) ausgesät und die Testsubstanzen werden in Form einer Konzentrationreihe zugegeben. Nach vier weiteren Tagen der Kultivierung in serumhaltigem Medium kann die Tumorzellproliferation/ Tumorzellvitalität mittels eines Alamarblue-Testsystem bestimmt werden.

**2.0 Versuchsdurchführung**

**2.1 Zellkultur**

Beispielsweise käuflich erhältliche Colon-Carcinom-Zelllinien, Zelllinien des Eierstocks, Zellinien der Prostata oder Zelllinien der Brust etc.
Die Zellen werden in Medium kultiviert. In Abständen von mehreren Tagen werden die Zellen mit Hilfe von Trypsin-Lösung von den Kulturschalen abgelöst und in geeigneten Verdünnung in frischem Medium ausgesät. Die Zellen werden bei 37° Celsius und 10% $CO_2$ kultiviert.

**2.2. Aussaat der Zellen**

Eine definierte Zellzahl (z.B. 2000 Zellen) werden pro Kultur/ well in einem Volumen von 180μl Kulturmedium in Mikrotiterplatten (96 well Zellkulturplatten) mit einer Mehrkanalpipette ausgesät. Die Zellen werden anschließend in einem CO2-Brutschrank (37°C und 10% CO2) kultiviert.

**2.3. Zugabe der Testsubstanzen**

Die Testsubstanzen werden beispielsweise in DMSO gelöst und anschließend in entsprechender Konzentration (gegebenenfalls einer Verdünnungsreihe) im Zellkulturmedium eingesetzt. Die Verdünnungsstufen können je nach Effizienz der Wirkstoffe und gewünschter Spreizung der Konzentrationen angepasst werden. Die Testsubstanzen werden in entsprechenden Konzentrationen mit Zellkulturmedium versetzt. Die Zugabe der Testsubstanzen zu den Zellen kann am selben Tag wie die Aussat der Zellen erfolgen. Dazu wird aus der Vorverdünnungsplatte jeweils 20μl Substanzlösung

in die Kulturen/wells gegeben. Die Zellen werden für weitere 4 Tage bei 37°Celsius und 10% $CO_2$ kultiviert.

**2.4. Messung der Farbreaktion**

Pro well werden jeweils 20μl AlamarBlue Reagenz gegeben und die Microtiterplatten werden beispielsweise für weitere sieben Stunden in einem CO2-Brutschrank (bei 37°C und 10% CO2) inkubiert. Die Platten werden an einem Reader mit einem Fluoreszenzfilter bei einer Wellenlänge von 540nm gemessen. Die Platten können direkt vor der Messung leicht geschüttelt werden.

**3. Auswertung**

Der Extinktionswert der Mediumkontrolle (keine Verwendung von Zellen und Testsubstanzen) wird von allen anderen Extinktionswerten subtrahiert. Die Kontrollen (Zellen ohne Testsubstanz) werden gleich 100 Prozent gesetzt und alle anderen Extinktionswerte hierzu in Beziehung gesetzt (beispielsweise in % der Kontrolle) ausgedrückt:

Rechnung:

$$\frac{100 * (\text{Wert mit Zellen und Testsubstanz - Wert der Mediumkontrolle})}{(\text{Wert mit Zellen - Wert der Mediumkontrolle})}$$

Die Bestimmung von $IC_{50}$ Werten (50%ige Hemmung) erfolgt mit Hilfe von Statistikprogrammen wie z.B. RS1. $IC_{50}$-Daten erfindungsgemäßer Verbindungen sind in Tabelle 1 angegeben.

**4.0 Test zur Inhibierung von PDK1**

Die Versuchsansätze werden in einem Flashplate-System mit 384 wells/Mikrotitrierplatte durchgeführt. Pro well werden jeweils die PDK1-Probe $His_6$-PDK1(Δ1-50)(3.4 nM), das PDK1-Substrat Biotin-bA-bA-KTFCGTPEYLAPEVRREP-RILSEEEQEMFRDFDYIADWC (400 nM), 4 μM ATP (mit 0.2μCi $^{33}$P-ATP/well) und die Testsubstanz in 50μl gebräuchlicher Versuchslösung für 60 Min bei 30°C inkubiert. Die Testsubstanzen werden in entsprechenden Konzentrationen (gegebenenfalls in einer Verdünnungsreihe) eingesetzt. Die Kontrolle wird ohne Testsubstanz durchgeführt. Die Reaktion wird mit gängigen Methoden gestoppt und gewaschen. Die Aktivität der Kinase wird über die eingebaute Radioaktivität im Topcount gemessen. Zur Bestimmung der unspezifischen Kinasereaktion (Leerwert) werden die Versuchsansätze in Gegenwart von 100 nM Staurosporin durchgeführt.

**5.0 Auswertung**

Die Radioaktivität (Zerfälle pro Minute) des Leerwerts (keine Verwendung von Testsubstanz in Gegenwart von Staurosporin) wird von allen anderen Radioaktivitätswerten substrahiert. Die Kontrollen (Kinaseaktivität ohne Testsubstanz) werden gleich 100 Prozent gesetzt und alle anderen Radioaktivitätswerte (nach Abzug des Leerwerts) hierzu in Beziehung gesetzt (beispielsweise in % der Kontrolle) ausgedrückt.

Rechnung:

$$\frac{100* (\text{Wert der Kinaseaktivität mit Testsubstanz - Leerwert})}{(\text{ Wert der Kontrolle - Leerwert})} = \text{\% der Kontrolle}$$

Die Bestimmung von $IC_{50}$ Werten (50%ige Hemmung) erfolgt mit Hilfe von Statistikprogrammen wie z.B. RS1. $IC_{50}$-Daten erfindungsgemäßer Verbindungen sind in Tabelle 1 angegeben.

| Material | Best. Nr. | Hersteller |
|---|---|---|
| Mikrotiterplatten für die Zellkultur (Nunclon Surface 96well Plate) | | 167008 Nunc |
| DMEM | P04-03550 | Pan Biotech |
| PBS (10x) Dulbecco | 14200-067 | Gibco |

(fortgesetzt)

| Material | Best. Nr. | Hersteller |
|---|---|---|
| 96well Platten (Polypropylen) | | 267334 Nunc |
| AlamarBlue™ | BUF012B | Serotec |
| FCS | 1302 | Pan Biotech GmbH |
| Trypsin/EDTA Solution 10x | L 2153 | Biochrom AG |
| 75cm$^2$ Kulturflaschen | 353136 | BD Falcon |
| A2780 | 93112519 | ECACC |
| Colo205 | CCL222 | ATCC |
| MCF7 | HTB22 | ATCC |
| PC3 | CRL-1435 | ATCC |
| 384well Flash Platten | SMP410A001PK | Perkin Elmer |

APCI-MS (atmospheric pressure chemical ionization - mass spectrometry) $(M+H)^+$.

HPLC-Gradientensystem

Säule:

ChromolithPerformance RP-18e (Merck KGaA, Cat. 1.02129.0001)

Eluenten:

Eluent A: 0.1 M wässr. NaH2PO4

Eluent B: Acetonitril + 10 % Wasser

Flußrate: 4 ml/min

Gradient:

0 min 1 % B

1 min 1 % B

7 min 99 % B

8 min 99 % B

Beispiel 1

Die Herstellung von 4-Butyl-6-(1H-pyrrolo[2,3-b]pyridin-3-yl)-pyrimidin-2-ylamin ("A1") erfolgt analog nachstehendem Schema

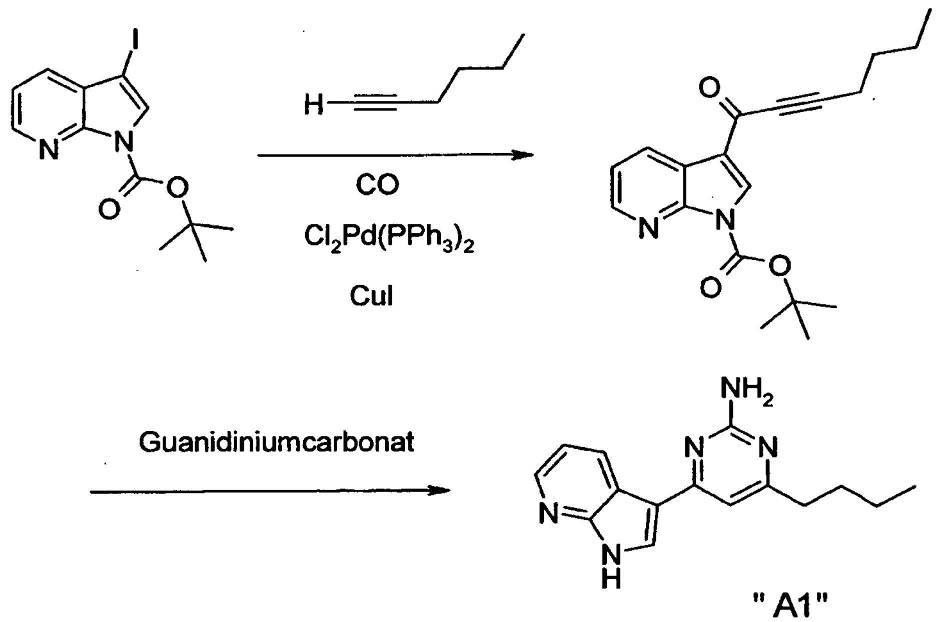


"A1"

1.1 Eine Lösung von 5.16 g (15.0 mmol) 3-Iod-pyrrolo[2,3-b]pyridin-1-carbonsäure-tert.-butylester in 100 ml Tetrahydrofuran wird mit 526 mg (0.75 mmol) Bis-(triphenylphosphin)-palladium(II)chlorid und 57.2 mg (0.30 mmol) Kupfer(I)iodid versetzt. In diese Lösung wird in einer Autoklavenapparatur Kohlenmonoxid eingeleitet. Dann werden 1.85 g (22.5 mmol) 1-Hexin und 1.52 g (15 mmol) Triethylamin zugegeben. Das Reaktionsgemisch wird in der Autoklavenapparatur 48 Stunden unter einem Druck von 1 atm Kohlenmonoxid gerührt. Das Reaktionsgemisch wird mit gesättigter Natriumchloridlösung gewaschen und die wässrige Phase mit Dichlormethan extrahiert. Die vereinigten organischen Phasen werden über Natriumsulfat getrocknet und eingedampft. Der Rückstand wird an einer Kieselgelsäule mit Petrolether/Ethylacetat als Laufmittel chromatographiert: 3-Hept-2-inoyl-pyrrolo[2,3-b]pyridin-1-carbonsäure-tert.-butylester als farbloses Öl; ESI 327.

1.2 Eine Lösung von 314 mg (0.96 mmol) 3-Hept-2-inoyl-pyrrolo[2,3-b]pyridin-1-carbonsäure-tert.-butylester und 130 mg (0.72 mmol) Guanidin-Carbonat in 2 ml Ethylenglycolmonomethylether wird mit 278 mg (2.0 mmol) Natriumcarbonat versetzt und das Gemisch 24 Stunden zum Sieden erhitzt. Nach dem Erkalten wird das Gemisch zwischen Wasser und Ethylacetat verteilt. Die organische Phase wird über Natriumsulfat getrocknet, eingedampft und der Rückstand mit präparativer HPLC chromatographiert. Man erhält 4-Butyl-6-(1H-pyrrolo[2,3-b]pyridin-3-yl)-pyrimidin-2-ylamin ("A1") als farblosen Feststoff; ESI 269;
$^1H$ NMR (DMSO-$d_6$) [ppm] 0.92 (t, J = 7.3 Hz, 3H), 1.35 (sextett, J = 7.3 Hz, 2H), 1.66 (quintett, J = 7.3 Hz, 2H), 2.49 (t, J = 7.3 Hz, 2H), 6.35 (s, 2H), 6.97 (s, 1H), 7.17 (dd, $J_1$ = 8.0 Hz, $J_2$ = 4.5 Hz, 1H), 8.27 (dd, $J_1$ = 4.5 Hz, $J_2$ = 1 Hz, 1 H), 8.30 (d, J = 3 Hz, 1 H), 8.90 (dd, $J_1$ = 4.5 Hz, $J_2$ = 8 Hz, $J_2$ = 1 Hz, 1H), 12.1 (bs, 1H).

Analog erhält man die nachstehenden Verbindungen

| Verbindung Nr. | Name und/oder Struktur | Analytische Daten |
|---|---|---|
| "A2" | 4-Phenyl-6-(1H-pyrrolo[2,3-b]pyridin-3-yl)-pyrimidin-2-ylamin | ESI 288 |
| "A3" | 4-Propyl-6-(1H-pyrrolo[2,3-b]pyridin-3-yl)-pyrimidin-2-ylamin | ESI 254 |
| "A4" | 4-Methoxymethyl-6-(1H-pyrrolo[2,3-b]pyridin-3-yl)-pyrimidin-2-ylamin | |

(fortgesetzt)

| Verbindung Nr. | Name und/oder Struktur | Analytische Daten |
|---|---|---|
| | 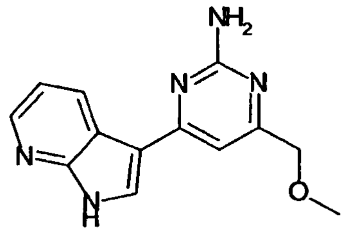 | |
| "A26" | 3-[2-Amino-6-(1H-pyrrolo[2,3-b]pyridin-3-yl)-pyrimidin-4-yl]-propan-1-ol 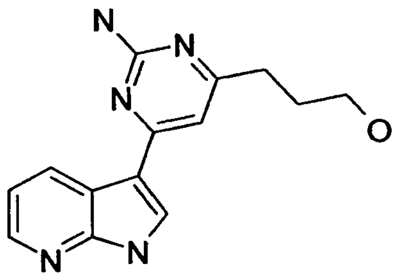 | ESI 270 |
| "A27" | 4-Methoxymethyl-6-(1H-pyrrolo[2,3-b]pyridin-3-yl)-pyrimidin-2-ylamin 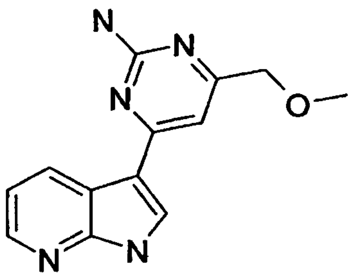 | ESI 256 |
| "A28" | 3-[2-Amino-6-(1H-pyrrolo[2,3-b]pyridin-3-yl)-pyrimidin-4-yl]-propionsäure 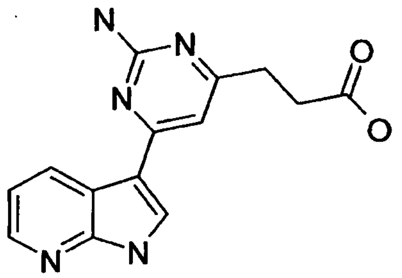 | ESI 284 |
| "A29" | 4-[2-Amino-6-(1H-pyrrolo[2,3-b]pyridin-3-yl)-pyrimidin-4-yl]-butan-1-ol 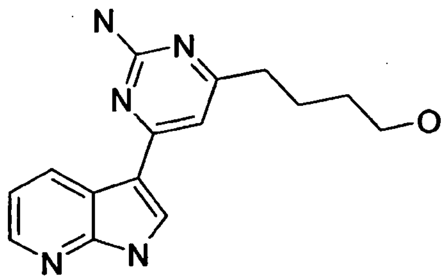 | ESI 284 |
| "A30" | 4-[2-Amino-6-(1H-pyrrolo[2,3-b]pyridin-3-yl)-pyrimidin-4-yl]-buttersäure | ESI 298 |

(fortgesetzt)

| Verbindung Nr. | Name und/oder Struktur | Analytische Daten |
|---|---|---|
| | 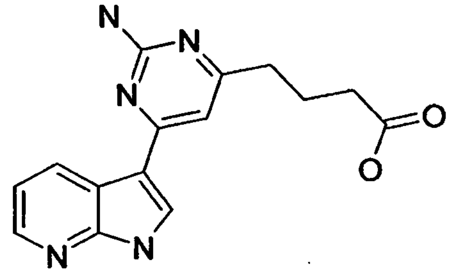 | |

Beispiel 2

Die Herstellung von 4-Ethyl-6-(1H-pyrrolo[2,3-b]pyridin-3-yl)-pyrimidin-2-ylamin ("A5") erfolgt analog nachstehendem Schema

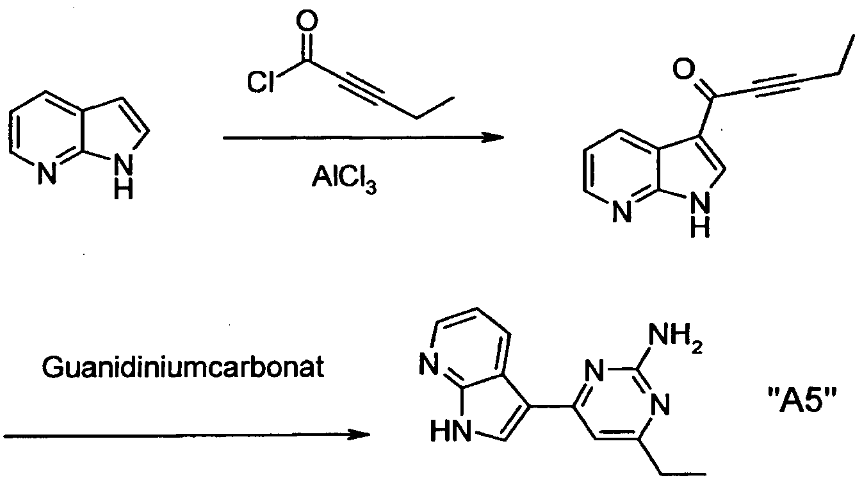

"A5"

2.1 Eine Lösung von 1.0 g (8.47 mmol) Pyrrolo[2,3-b]pyridin in 100 ml Dichlormethan wird mit 5.6 g (42.0 mmol) Aluminiumtrichlorid versetzt und 2 h bei Raumtemperatur gerührt. Danach werden 52 ml (0.2 M, 10.09 mmol) Pent-2-inoylchlorid gelöst in Dichlormethan zur Reaktionslösung langsam zugetropft. Man lässt 16 h bei Raumtemperatur weiterrühren. Das Reaktionsgemisch wird mit gesättigter Natriumhydrogencarbonatlösung bis zur Neutralreaktion versetzt und der Aluminiumsalzniederschlag abgesaugt. Die wässrige Phase wird abgetrennt und zweimal mit Dichlormethan extrahiert. Die vereinigten organischen Phasen werden mit gesättigter Natriumchloridlösung gewaschen, über Magnesiumsulfat getrocknet und eingedampft. Der Rückstand wird ohne weitere Aufreinigung in der nächsten Reaktion eingesetzt. Man erhält 1-(1H-Pyrrolo[2,3-b]pyridin-3-yl)-pent-2-in-1-on (74%ige Reinheit) als farbloses Öl; ESI 199.

2.2 Eine Lösung von 600 mg (3.03 mmol) (1 H-Pyrrolo[2,3-b]pyridin-3-yl)-pent-2-in-1-on und 660 mg (3.66 mmol) Guanidin-Carbonat in 10 ml Ethylenglycolmonomethylether wird mit 2.1 g (15.12 mmol) Kaliumcarbonat versetzt und das Gemisch 4 Stunden zum Sieden erhitzt. Nach dem Erkalten wird das Gemisch zwischen Wasser und Ethylacetat verteilt. Die organische Phase wird über Natriumsulfat getrocknet, eingedampft und der Rückstand mit präparativer HPLC chromatographiert. Man erhält 4-Ethyl-6-(1H-pyrrolo[2,3-b]pyridin-3-yl)-pyrimidin-2-ylamin ("A5") als farblosen Feststoff; ESI 240;
$^1$H NMR (DMSO-$d_6$) δ [ppm] 1.22 (t, J = 7.56 Hz, 3H), 2.50 (dq, $J_1$ = 4.0 Hz, $J_2$ = 1 Hz, 2H), 6.38 (s, 2H), 6.97 (s, 1H), 7.17 (dd, $J_1$ = 8.14 Hz, $J_2$ = 4.68 Hz, 1 H), 8.27 (dd, $J_1$ = 4.9 Hz, $J_2$ = 1.73 Hz, 1 H), 8.32 (d, J = 2.89 Hz, 1H), 8.91 (dd, $J_1$ = 7.95 Hz, $J_2$ = 1.56 Hz, 1H), 12.12 (bs, 1H).

Analog erhält man die nachstehenden Verbindungen

| Verbindung Nr. | Name und/oder Struktur | Analytische Daten |
|---|---|---|
| "A6" | 4-Pentyl-6-(1H-pyrrolo[2,3-b]pyridin-3-yl)-pyrimidin-2-ylamin | ESI 282 |
| "A8" | 4-(4-Propyl-cyclohexyl)-6-(1H-pyrrolo[2,3-b]pyridin-3-yl)-pyrimidin-2-ylamin 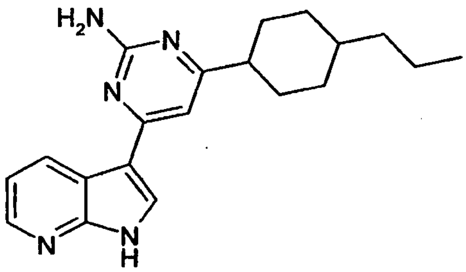 | ESI 336 |
| "A9" | 4-(4-Propyl-phenyl)-6-(1H-pyrrolo[2,3-b]pyridin-3-yl)-pyrimidin-2-ylamin | ESI 330 |
| "A10" | 4-Methyl-6-(1H-pyrrolo[2,3-b]pyridin-3-yl)-pyrimidin-2-ylamin | |
| "A31" | 4-Methyl-6-(1H-pyrrolo[2,3-b]pyridin-3-yl)-pyrimidin-2-ylamin 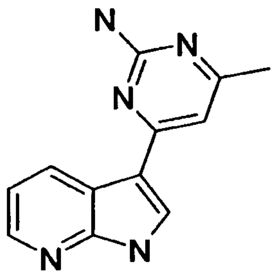 | ESI 226 |
| "A32" | 4-(5-Bromo-1H-pyrrolo[2,3-b]pyridin-3-yl)-6-ethyl-pyrimidin-2-ylamin | ESI 319 |
| | 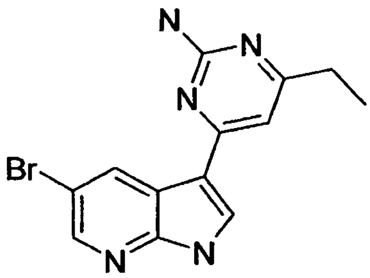 | |
| "A33" | 4-(3-Nitro-phenyl)-6-(1H-pyrrolo[2,3-b]pyridin-3-yl)-pyrimidin-2-ylamin 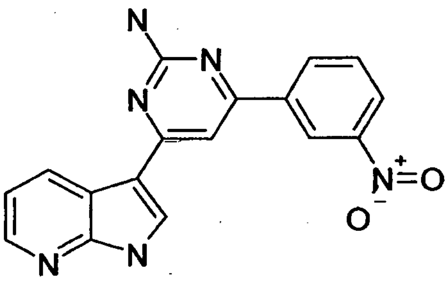 | ESI 333 |
| "A34" | 4-(3-Amino-phenyl)-6-(1H-pyrrolo[2,3-b]pyridin-3-yl)-pyrimidin-2-ylamin | ESI 303 |

(fortgesetzt)

| Verbindung Nr. | Name und/oder Struktur | Analytische Daten |
|---|---|---|
| | 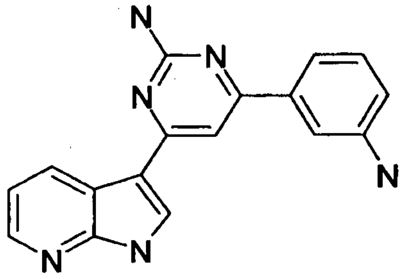 | |
| "A35" | 4-Ethyl-6-(2-methyl-1H-pyrrolo[2,3-b]pyridin-3-yl)-pyrimidin-2-ylamin 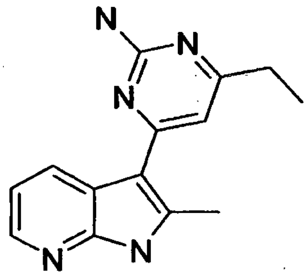 | ESI 254 |
| "A36" | 4-(4-Chloro-1H-pyrrolo[2,3-b]pyridin-3-yl)-6-ethyl-pyrimidin-2-ylamin | ESI 274 |
| | 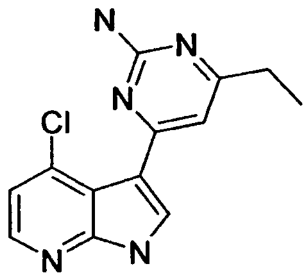 | |
| "A37" | 3-(2-Amino-6-ethyl-pyrimidin-4-yl)-1H-pyrrolo[2,3-b]pyridine-5-carbonitril 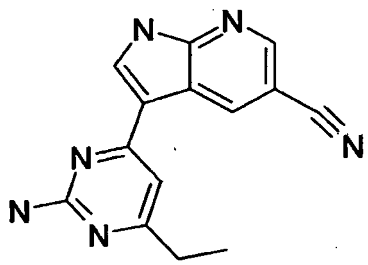 | ESI 265 |

Beispiel 3

Die Herstellung von 4-(1-Benzyl-1 H-pyrrolo[2,3-b]pyridin-3-yl)-6-butyl-pyrimidin-2-ylamin ("A7") erfolgt analog nachstehendem Schema

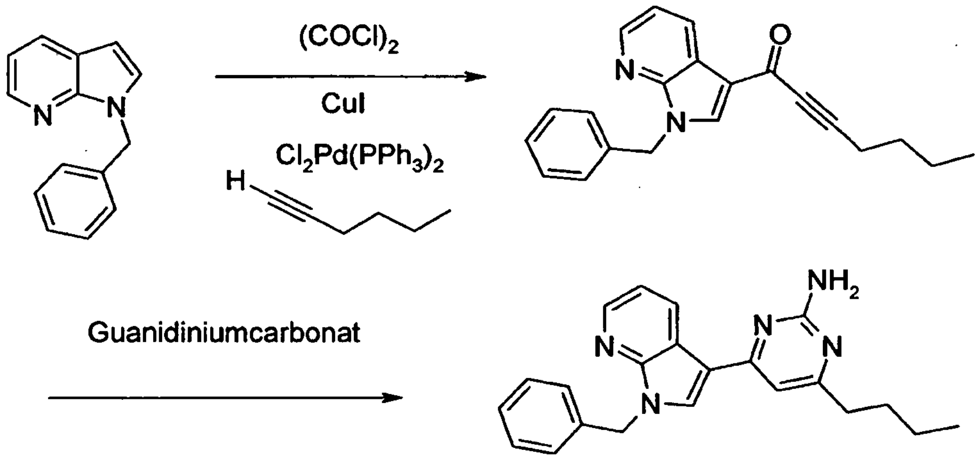


"A7"

3.1 1.04 g (5.00 mmol) N-Benzyl-7-azaindol werden unter Stickstoff in 25 ml 1,2-Dimethoxyethan gelöst und auf 0° C abgekühlt. Dann werden 0.44 ml (5.00 mmol) Oxalylchlorid bei 0° C zugetropft und die Reaktionsmischung 2 Stunden zum Sieden erhitzt. Danach werden 35 mg (0.05 mmol) Bis-(triphenylphosphin)-palladium(II)chlorid, 10 mg (0.05 mmol) Kupfer(I)-iodid, 0.59 ml (5.00 mmol) 1-Hexin und 1.38 ml (10.0 mmol) Triethylamin nacheinander zugegeben und die Reaktionsmischung 1 Stunde bei Raumtemperatur gerührt. Das Reaktionsgemisch wird mit gesättigter Natriumchloridlösung verdünnt und mit 20 ml Dichlormethan extrahiert. Die vereinigten organischen Phasen werden über Natriumsulfat getrocknet und eingedampft. Der Rückstand wird an einer Kieselgelsäule mit Petrolether/Ethylacetat chromatographiert: 1-(1-Benzyl-1H-pyrrolo[2,3-b]pyridin-3-yl)-hept-2-in-1-on als orangegelbes Öl; ESI 317.

3.2 Die Cyclisierung mit Guanidiniumcarbonat zu 4-(1-Benzyl-1 H-pyrrolo[2,3-b]pyridin-3-yl)-6-butyl-pyrimidin-2-ylamin ("A7") (farblose Kristalle; ESI 358) wird analog zu Beispiel 1 durchgeführt.

Analog erhält man die nachstehenden Verbindungen

| Verbindung Nr. | Name und/oder Struktur | Analytische Daten |
|---|---|---|
| "A7a" | 4-[1-(4-Methoxybenzyl)-1H-pyrrolo[2,3-b]pyridin-3-yl]-6-methoxymethyl-pyrimidin-2-ylamin 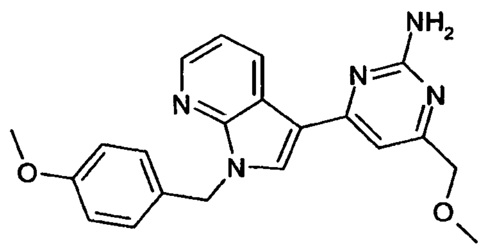 | ESI 376 |
| "A38" | [2-Amino-6-(1-benzyl-1H-pyrrolo[2,3-b]pyridin-3-yl)-pyrimidin-4-yl]-methanol | ESI 332 |

(fortgesetzt)

| Verbindung Nr. | Name und/oder Struktur | Analytische Daten |
|---|---|---|
| | 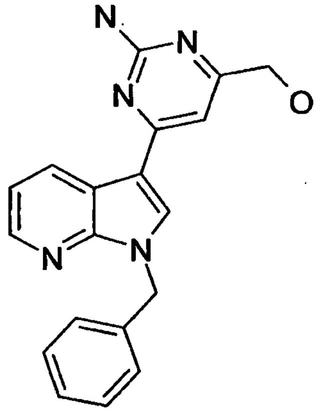 | |

Beispiel 4

Die Herstellung von 4-Hydroxymethyl-6-(1H-pyrrolo[2,3-b]pyridin-3-yl)-pyrimidin-2-ylamin ("A11") erfolgt analog nachstehendem Schema

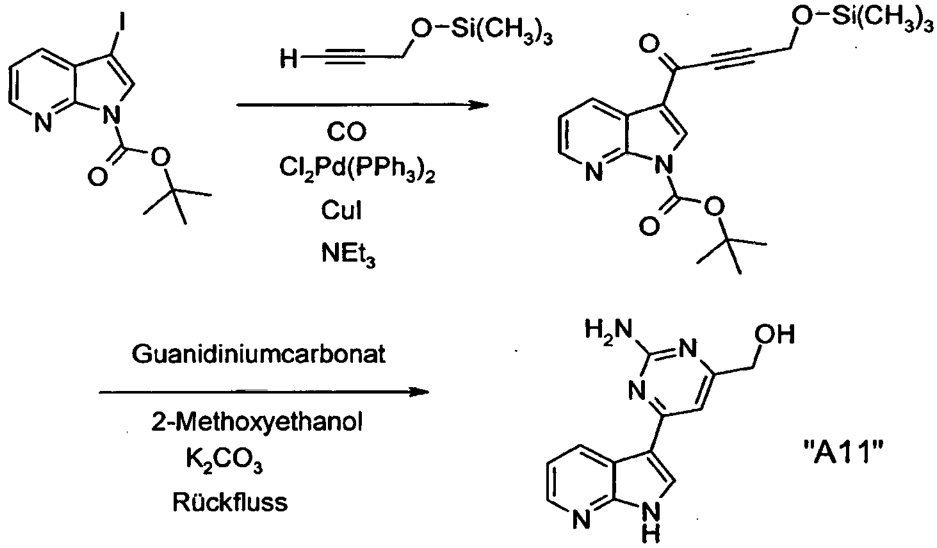


"A11"

Analog erhält man die nachstehenden Verbindungen

| Verbindung Nr. | Name und/oder Struktur | Analytische Daten |
|---|---|---|
| "A26" | 3-[2-Amino-6-(1H-pyrrolo[2,3-b]pyridin-3-yl)-pyrimidin-4-yl]-propan-1-ol | ESI 270 |

(fortgesetzt)

| Verbindung Nr. | Name und/oder Struktur | Analytische Daten |
|---|---|---|
| | 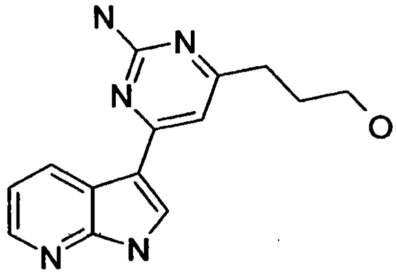 | |
| "A29" | 4-[2-Amino-6-(1H-pyrrolo[2,3-b]pyridin-3-yl)-pyrimidin-4-yl]-butan-1-ol 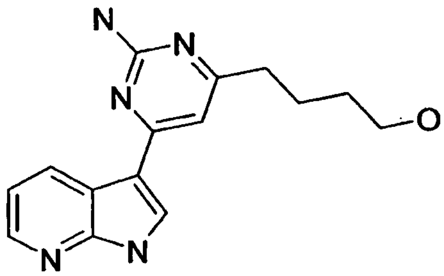 | ESI 284 |

Beispiel 5

Die Herstellung von 2-[2-Amino-6-(1H-pyrrolo[2,3-b]pyridin-3-yl)-pyrimidin-4-ylmethyl]isoindol-1,3-dion ("A12"),
4-Aminomethyl-6-(pyrrolo[2,3-b]pyridin-3-yl)-pyrimidin-2-ylamin ("A15) und 4-(N,N-Dimethylaminomethyl)-6-(pyrrolo[2,3-b]pyridin-3-yl)-pyrimidin-2-ylamin ("A18")
erfolgt analog nachstehendem Schema

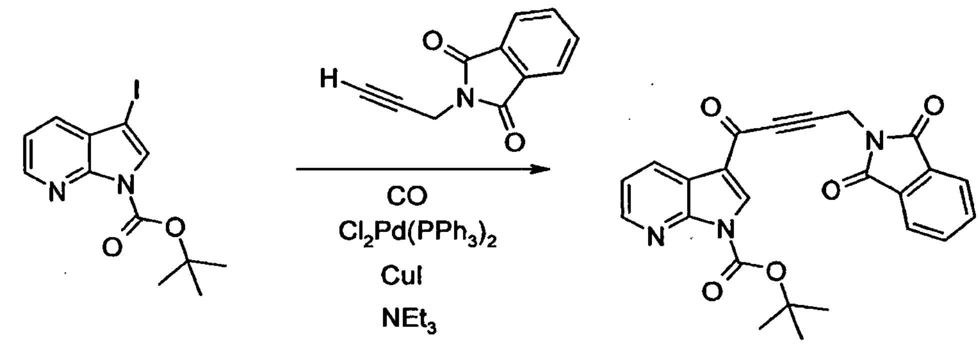


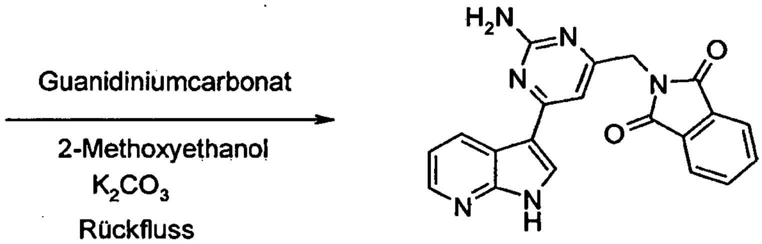


"A12"

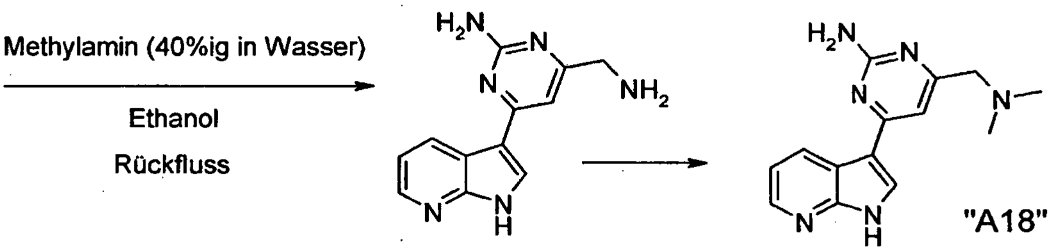


"A15" "A18"

Analog erhält man die nachstehenden Verbindungen

| Verbindung Nr. | Name und/oder Struktur | Analytische Daten |
|---|---|---|
| "A13" | 2-{3-[2-Amino-6-(1H-pyrrolo[2,3-b]pyridin-3-yl)-pyrimidin-4-yl]-propyl} -isoindol-1,3-dion 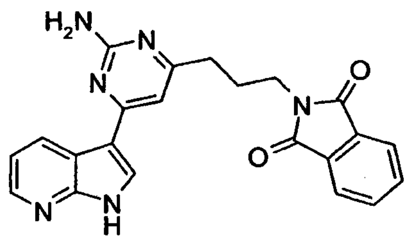 | ESI 399 |
| "A14" | 2-{4-[2-Amino-6-(1H-pyrrolo[2,3-b]pyridin-3-yl)-pyrimidin-4-yl]-butyl} -isoindol-1,3-dion 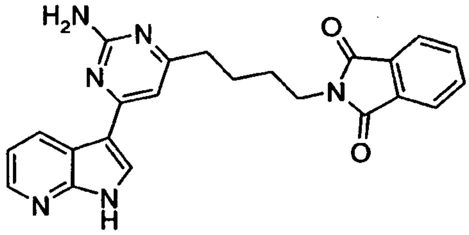 | ESI 413 |

(fortgesetzt)

| Verbindung Nr. | Name und/oder Struktur | Analytische Daten |
|---|---|---|
| "A16" | 4-(3-Amino-propyl)-6-(1H-pyrrolo[2,3-b]pyridin-3-yl)-pyrimidin-2-ylamin 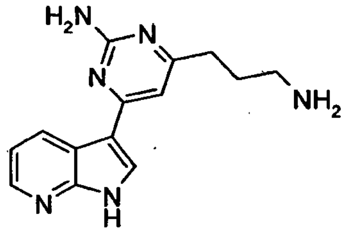 | ESI 269 |
| "A17" | 4-(4-Amino-butyl)-6-(1H-pyrrolo[2,3-b]pyridin-3-yl)-pyrimidin-2-ylamin 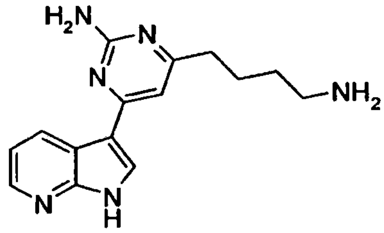 | ESI 283 |
| "A19" | 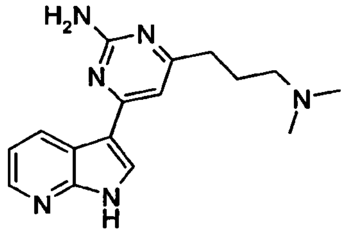 | |

Beispiel 6

Die Herstellung von 4-(Pyrrolidin-1-yl-propyl)-6-(1H-pyrrolo[2,3-b]pyridin-3-yl)-pyrimidin-2-ylamin ("A20") erfolgt analog nachstehendem Schema

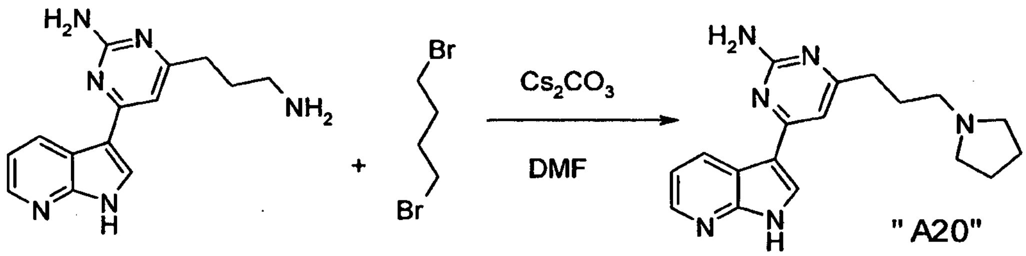


"A20"

Beispiel 7

Die Herstellung von 4-(Piperidin-4-yl-methyl)-6-(1H-pyrrolo[2,3-b]pyridin-3-yl)-pyrimidin-2-ylamin ("A21") und 4-(1-Methyl-piperidin-4-yl-methyl)-6-(1H-pyrrolo[2,3-b]pyridin-3-yl)-pyrimidin-2-ylamin ("A22") erfolgt analog nachstehendem Schema

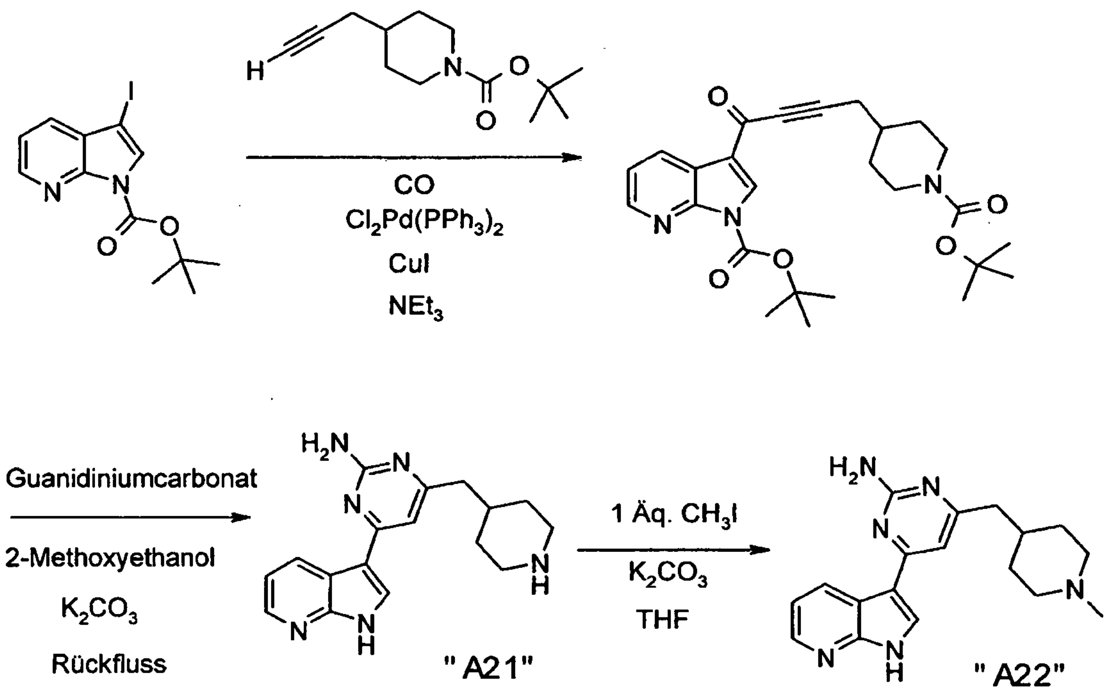


"A21"

"A22"

Die Herstellung des Zwischenprodukts 4-Prop-2-inyl-piperidin-1-carbonsäure-tert.-butylester erfolgt wie in WO00/59503 (Seite 215) beschrieben.

Analog erhält man die nachstehenden Verbindungen

| Verbindung Nr. | Name und/oder Struktur | Analytische Daten |
|---|---|---|
| "A23" | 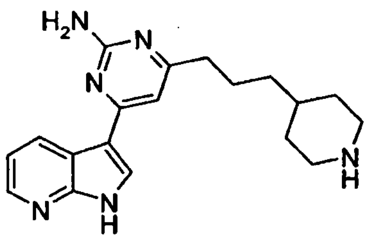 | |
| "A24" | 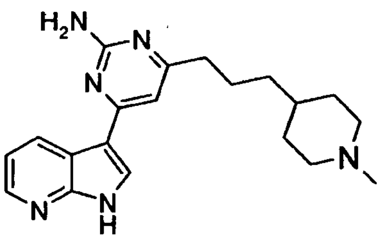 | |
| "A25" | 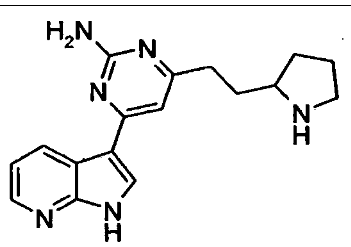 | |
| "A39" | 4-(3-Piperazin-1-yl-propyl)-6-(1H-pyrrolo[2,3-b]pyridin-3-yl)-pyrimidin-2-ylamin | ESI 338 |

(fortgesetzt)

| Verbindung Nr. | Name und/oder Struktur | Analytische Daten |
|---|---|---|
| | 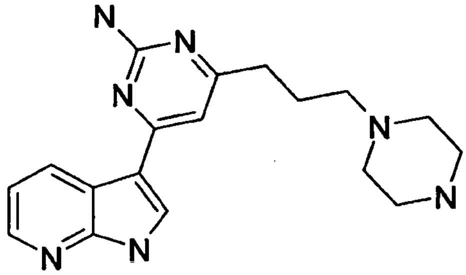 | |
| "A40" | 4-(3-Morpholin-4-yl-propyl)-6-(1H-pyrrolo[2,3-b]pyridin-3-yl)-pyrimidin-2-ylamin 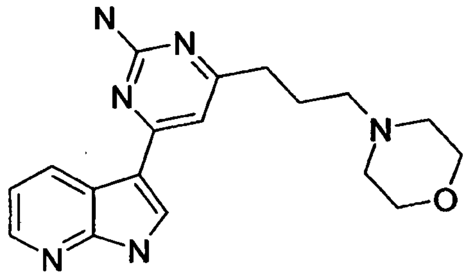 | ESI 339 |
| "A41" | 4-[3-(4-Methyl-piperazin-1-yl)-propyl]-6-(1H-pyrrolo[2,3-b]pyridin-3-yl)-pyrimidin-2-ylamin 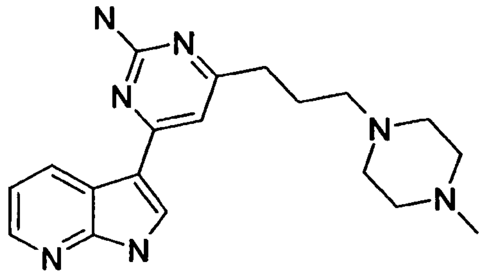 | ESI 352 |

Beispiel 8

Die Herstellung von 4-Ethyl-6-{5-[1-(1-methyl-piperidin-4-yl)-1H-pyrazol-4-yl]-1H-pyrrolo[2,3-b]pyridin-3-yl}-pyrimidin-2-ylamin erfolgt nach dem nachfolgenden Schema

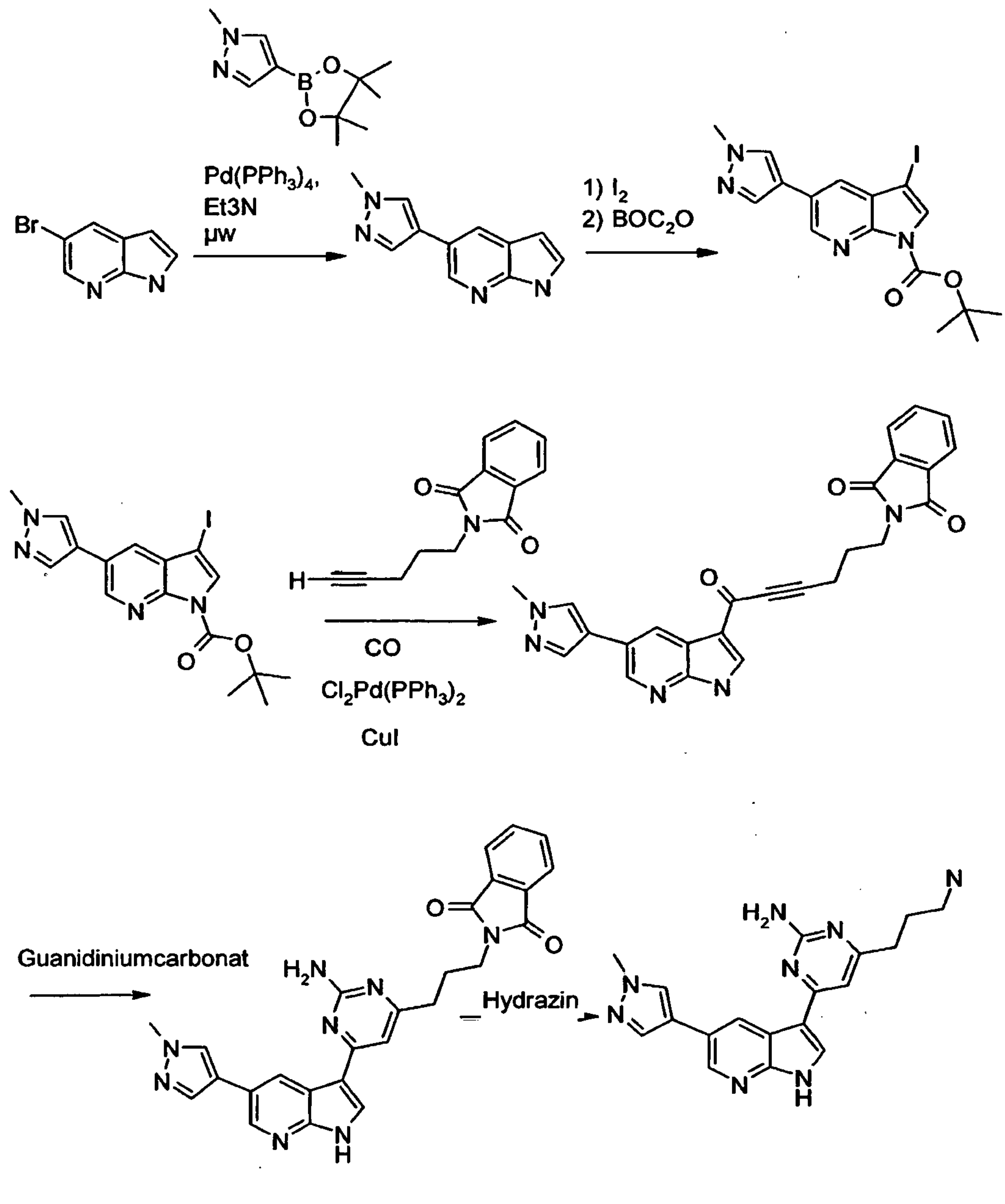


"A42"

8.1 Eine Suspension von 1.00 g (5.0 mmol) 5-Bromo-7-azaindol, 2.00 g (9.1 mmol) 1-Methylpyrazol-4-Boronsäure pinacol ester, 300 mg (0.26 mmol) $Pd[(PPh_3]_4$ und 9 ml $Na_2CO_3$ (Zugabe als 2 M-Lösung) in 12 ml DMF wird 30 min bei 120 °C unter Mikrowellenbedingungen erhitzt. Das Reaktionsgemisch wird filtriert und das Filtrat eingedampft. Der Rückstand wird an einer Kieselgelsäule mit Dichlormethan/ Methanol als Laufmittel aufgereinigt. Man erhält 5-(1-Methyl-1H-pyrazol-4-yl)-1H-pyrrolo[2,3-b]pyridine als gelben Feststoff, ESI 199

8.2 Zu einer Suspension von 901 mg (4.4 mmol) 5-(1-Methyl-1H-pyrazol-4-yl)-1H-pyrrolo[2,3-b]pyridin und 620 mg (11.1 mmol) KOH in 12 ml DMF wird bei RT eine Lösung von 1.125 g (4.4 mmol) Iod in 16 ml DMF langsam zugetropft. Man rührt nach beendeter Zugabe noch 30 min bei RT. Anschließend wird das Reaktionsgemisch auf 300 ml einer eiskalten, gesättigten Natriumsulfit Lösung gegossen. Der gelbe Niederschlag wird abgesaugt und mit 10 ml eiskaltem Wasser gewaschen. Der Filterrückstand wird getrocknet. Man erhält 3-Iodo-5-(1-methyl-1H-pyrazol-4-yl)-1 H-pyrrolo[2,3-b]pyridin als beige Kristalle, ESI 325

8.3 Zu einer Lösung von 238 mg (0.736 mmol) 3-Iodo-5-(1-methyl-1H-pyrazol-4-yl)-1H-pyrrolo[2,3-b]pyridin in 2 ml Dichlormethan gibt man 0.3 ml (2.28 mmol) Triethylamin und 9 mg (0.07 mmol) 4-(Dimethylamino)-pyridin zu.

Anschließend tropft man eine Lösung von 0.158 ml (0783 mmol) Di-tert-butyldicarbonat in 2.5 ml Dichlormethan zu der Reaktionslösung zu. Man rührt 16h bei RT. Das Reaktionsgemisch wird zweimal mit 5 ml gesättigter Natriumsulfitlösung und einmal mit Wasser extrahiert. Die vereinigten organischen Phasen werden über $MgSO_4$ getrocknet, der Feststoff abgetrennt und das Lösungsmittel im Vakuum entfernt. Der Rückstand wird an einer Kieselgelsäule mit Cyclohexan/Ethylacetat als Laufmittel aufgetrennt. Man erhält 3-Iodo-5-(1-methyl-1H-pyrazol-4-yl)-pyrrolo[2,3-b]pyridin-1-carbonsäure tert-butyl ester als braune gelförmige Substanz, ESI 425

8.4 Eine Lösung von 417 mg (0.9 mmol) 5-Metylpyrazolo-3-Iod-pyrrolo[2,3-b]pyridin-1-carbonsäure-tert.-butylester in 7.5 ml Tetrahydrofuran wird mit 69 mg (0.10 mmol) Bis-(triphenylphosphin)-palladium(II)chlorid und 19 mg (0.10 mmol) Kupfer(I)iodid versetzt. In diese Lösung wird in einer Autoklavenapparatur Kohlenmonoxid eingeleitet. Dann werden 315 mg (0.145 mmol) 2-Pent-4-ynyl-isoindol-1,3-dion und 0.137 ml (0.925 mmol) Triethylamin zugegeben. Das Reaktionsgemisch wird in der Autoklavenapparatur 4 Tage unter einem Druck von 5 bar Kohlenmonoxid gerührt. Das Reaktionsgemisch wird im Vakuum eingeengt und der Rückstand per präparativer HPLC gereinigt: Man erhält 2-{6-[5-(1-Methyl-1H-pyrazol-4-yl)-1H-pyrrolo[2,3-b]pyridin-3-yl]-6-oxo-hex-4-ynyl}-isoindol-1,3-dion als gelben Feststoff; ESI 438

8.5 Eine Lösung von 116 mg (0.265 mmol) 2-{6-[5-(1-Methyl-1H-pyrazol-4-yl)-1H-pyrrolo[2,3-b]pyridin-3-yl]-6-oxo-hex-4-ynyl}-isoindol-1,3-dion und 70 mg (0.39 mmol) Guanidin-Carbonat in 4 ml Ethylenglycolmonomethylether wird mit 0.92 ml (6.7 mmol) Triethylamin versetzt und das Gemisch 6 Stunden zum Sieden erhitzt. Nach dem Erkalten wird das Gemisch eingedampft und der Rückstand per präparativer HPLC aufgereinigt. Man erhält 2-(3-{2-Amino-6-[5-(1-methyl-1H-pyrazol-4-yl)-1H-pyrrolo[2,3-b]pyridin-3-yl]-pyrimidin-4-yl}-propyl)-isoindol-1,3-dion als braunen Feststoff; ESI 479, EMD 1183586.

8.6 Zu einer Lösung von 29 mg (0.06 mmol) 2-(3-{2-Amino-6-[5-(1-methyl-1H-pyrazol-4-yl)-1H-pyrrolo[2,3-b]pyridin-3-yl]-pyrimidin-4-yl}-propyl)-isoindol-1,3-dion in 0.5 ml Methanol wird 0.003 ml (0.06 mmol) Hydraziniumhydroxid bei RT zugegeben. Man erhitzt die Reaktionslösung in der Mikrowelle für 5 min bei 60°C. Das Reaktionsgemisch wird im Vakuum eingedampft und der Rückstand per präparativer HPLC gereinigt. Man erhält 4-(3-Amino-propyl)-6-[5-(1-methyl-1H-pyrazol-4-yl)-1 H-pyrrolo[2,3-b]pyridin-3-yl]-pyrimidin-2-ylamin als gelben Feststoff, ESI 349

Analog erhält man nachfolgende Verbindungen

| Verbindung Nr. | Name und/oder Struktur | Analytische Daten |
|---|---|---|
| "A43" | 4-(3-Amino-propyl)-6-(5-furan-2-yl-1H-pyrrolo[2,3-b]pyridin-3-yl)-pyrimidin-2-ylamin 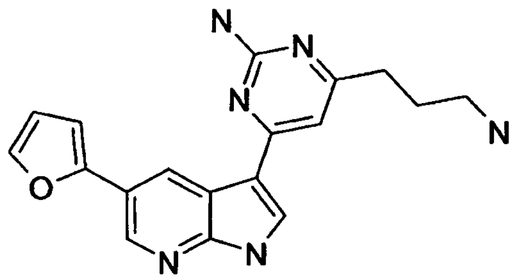 | ESI 335 |
| "A44" | 4-(3-Amino-propyl)-6-[5-(1-methyl-1H-pyrazol-4-yl)-1H-pyrrolo[2,3-b]pyridin-3-yl]-pyrimidin-2-ylamin 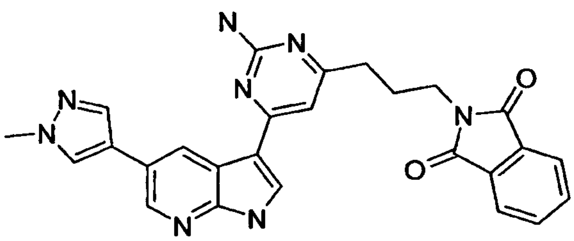 | ESI 479 |

Beispiel 9

Die Herstellung von 4-Ethyl-6-[5-(1-methyl-1H-pyrazol-4-yl)-1H-pyrrolo[2,3-b]pyridin-3-yl]-pyrimidin-2-ylamin erfolgt nach dem folgendem Schema

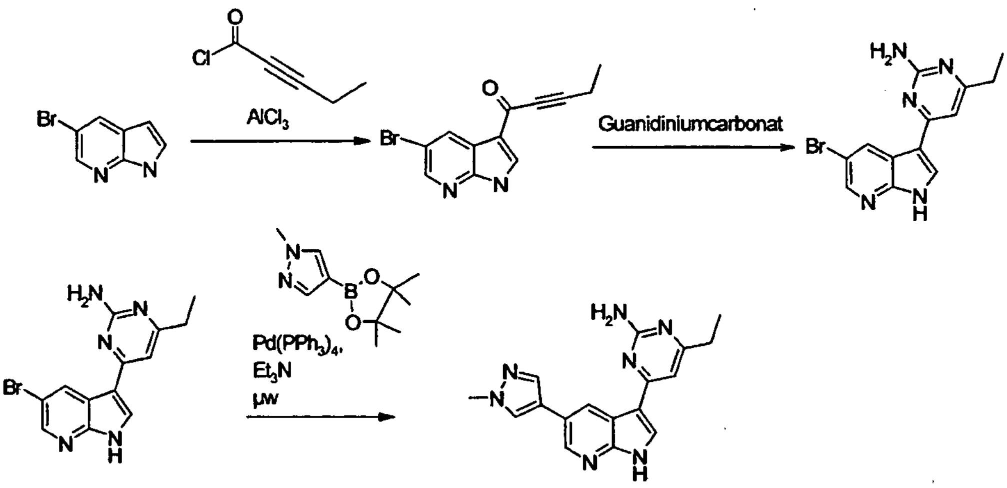


"A45"

Die Herstellung von EMD 1126600 erfolgt analog zu Beispiel 2.

Eine Suspension von 50 mg (0.157 mmol) 4-(5-Bromo-1H-pyrrolo[2,3-b]pyridin-3-yl)-6-ethyl-pyrimidin-2-ylamin, 56 mg (0.282 mmol) Methylpyrazol-4-Boronsäure pinacol ester, 9 mg (0.008 mmol) Tetrakis-(triphenylphosphin)-palladium(0) und 0.45 ml einer 2M $Na_2CO_3$-Lösung (0.900 mmol) wird in 0.6 ml DMF für 30 min bei 120°C in der Mikrowelle erhitzt. Das Reaktionsgemisch wird filtriert, der Filterkuchen mit MeOH gewaschen und das Filtrat im Vakuum eingedampft. Der Rückstand wird über präparative HPLC gereinigt. Man erhält 4-Ethyl-6-[5-(1-methyl-1H-pyrazol-4-yl)-1 H-pyrrolo[2,3-b]pyridin-3-yl]-pyrimidin-2-ylamin als einen farblosen Feststoff, ESI 320.

Analog erhält man nachfolgende Verbindungen

| Verbindung Nr. | Name und/oder Struktur | Analytische Daten |
|---|---|---|
| "A46" | 4-Ethyl-6-(5-pyridin-3-yl-1H-pyrrolo[2,3-b]pyridin-3-yl)-pyrimidin-2-ylamin 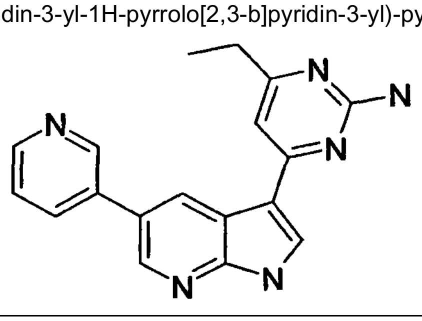 | ESI 317 |
| "A47" | 4-Ethyl-6-(5-pyridin-4-yl-1H-pyrrolo[2,3-b]pyridin-3-yl)-pyrimidin-2-ylamin | ESI 317 |

(fortgesetzt)

| Verbindung Nr. | Name und/oder Struktur | Analytische Daten |
|---|---|---|
| | 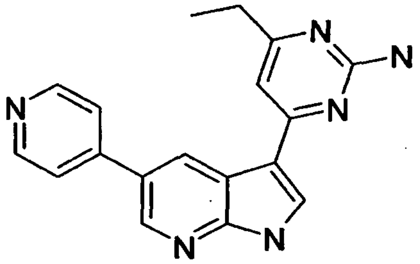 | |
| "A48" | 4-[5-(6-Amino-pyridin-3-yl)-1H-pyrrolo[2,3-b]pyridin-3-yl]-6-ethyl-pyrimidin-2-ylamin 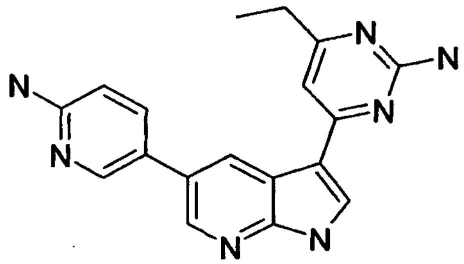 | ESI 332 |
| "A49" | 4-Ethyl-6-(5-furan-2-yl-1H-pyrrolo[2,3-b]pyridin-3-yl)-pyrimidin-2-ylamin 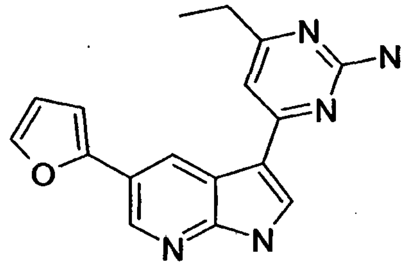 | ESI 306 |
| "A50" | 3-Amino-5-[3-(2-amino-6-ethyl-pyrimidin-4-yl)-1H-pyrrolo[2,3-b]pyridin-5-yl]-benzoesäure 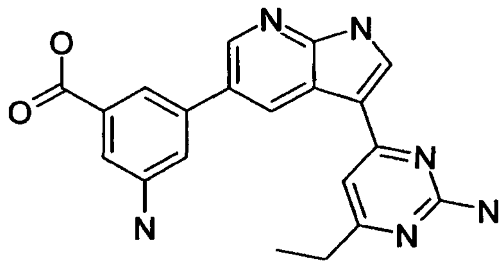 | ESI 375 |
| "A51" | 4-[5-(3,5-Dimethyl-isoxazol-4-yl)-1H-pyrrolo[2,3-b]pyridin-3-yl]-6-ethyl-pyrimidin-2-ylamin 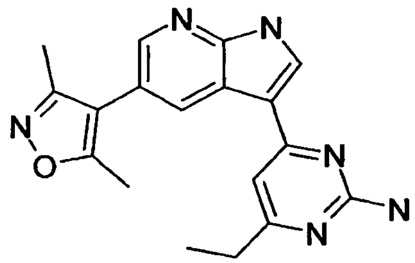 | ESI 335 |
| "A52" | 4-Ethyl-6-(5-phenyl-1H-pyrrolo[2,3-b]pyridin-3-yl)-pyrimidin-2-ylamin | ESI 316 |

(fortgesetzt)

| Verbindung Nr. | Name und/oder Struktur | Analytische Daten |
|---|---|---|
| | 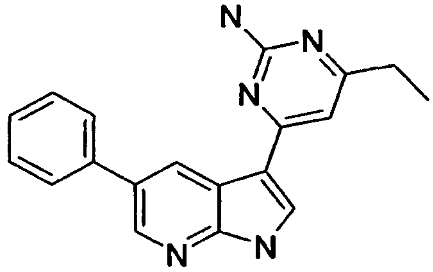 | |
| "A53" | 4-[5-(6-Dimethylamino-pyridin-3-yl)-1H-pyrrolo[2,3-b]pyridin-3-yl]-6-ethyl-pyrimidin-2-ylamin 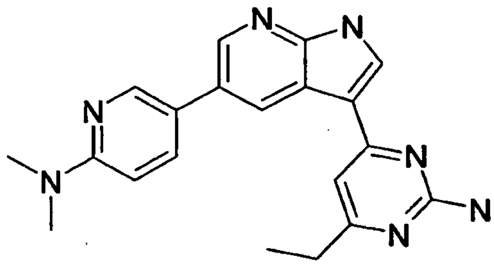 | ESI 360 |
| "A54" | 3-[3-(2-Amino-6-ethyl-pyrimidin-4-yl)-1H-pyrrolo[2,3-b]pyridin-5-yl]-benzonitril 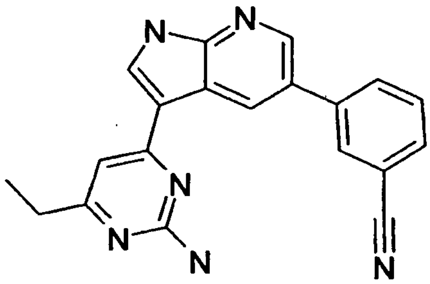 | ESI 341 |
| "A55" | 4-Ethyl-6-{5-[1-(3-methoxy-propyl)-1H-pyrazol-4-yl]-1H-pyrrolo[2,3-b]pyridin-3-yl}-pyrimidin-2-ylamin 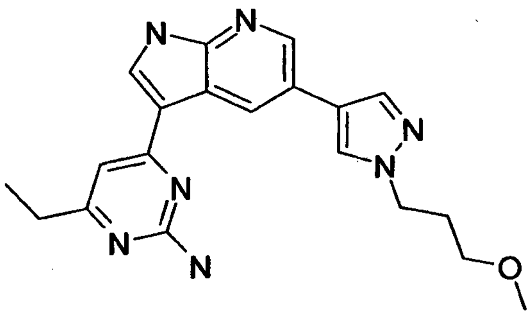 | ESI 378 |
| "A56" | 4-Ethyl-6-{5-[1-(2-methoxy-ethyl)-1H-pyrazol-4-yl]-1H-pyrrolo[2,3-b]pyridin-3-yl}-pyrimidin-2-ylamin | ESI 364 |

(fortgesetzt)

| Verbindung Nr. | Name und/oder Struktur | Analytische Daten |
|---|---|---|
| | 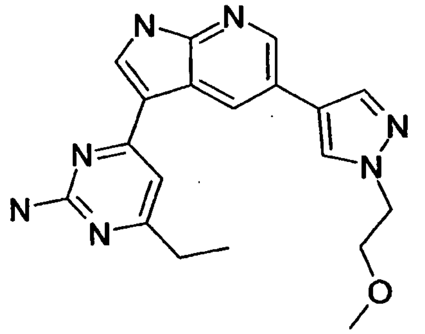 | |
| "A57" | 4-Ethyl-6-[5-(6-fluoro-4-methyl-pyridin-3-yl)-1H-pyrrolo[2,3-b]pyridin-3-yl]-pyrimidin-2-ylamin 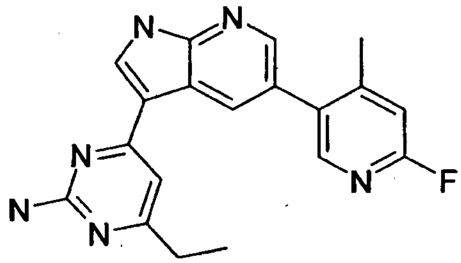 | ESI 349 |
| "A58" | 5-[3-(2-Amino-6-ethyl-pyrimidin-4-yl)-1H-pyrrolo[2,3-b]pyridin-5-yl]-pyridin-2-carbonsäure methylamid 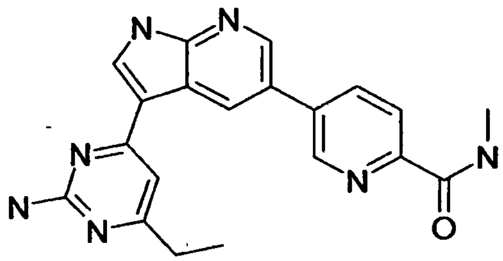 | ESI 374 |
| "A59" | 4-Ethyl-6-{5-[1-(3-Methyl-butyl)-1H-pyrazol-4-yl]-1H-pyrrolo[2,3-b]pyridin-3-yl}-pyrimidin-2-ylamin 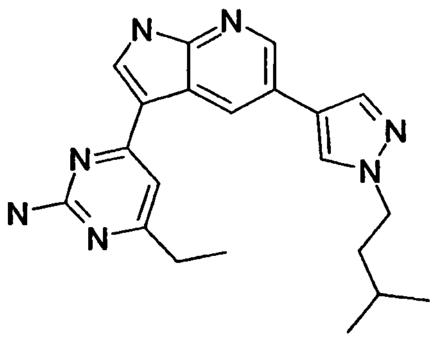 | ESI 376 |
| "A60" | 4-Ethyl-6-[5-(1H-pyrazol-4-yl)-1H-pyrrolo[2,3-b]pyridin-3-yl]-pyrimidin-2-ylamin | ESI 306 |

(fortgesetzt)

| Verbindung Nr. | Name und/oder Struktur | Analytische Daten |
|---|---|---|
| | 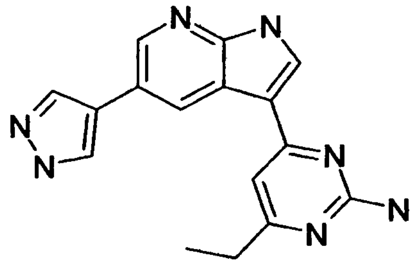 | |
| "A61" | 4-Ethyl-6-[5-(2H-pyrazol-3-yl)-1H-pyrrolo[2,3-b]pyridin-3-yl]-pyrimidin-2-ylamin 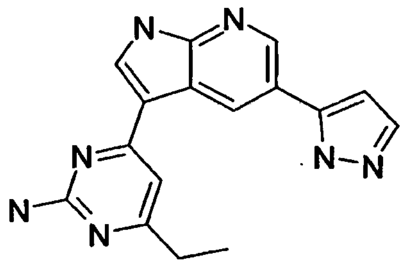 | ESI 306 |
| "A62" | 4-Ethyl-6-[5-(2-methyl-2H-pyrazol-3-yl)-1H-pyrrolo[2,3-b]pyridin-3-yl]-pyrimidin-2-ylamin 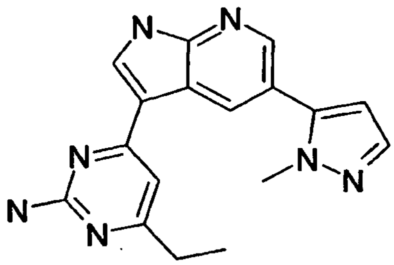 | ESI 320 |
| "A63" | 4-Ethyl-6-[5-(1-isopropyl-1H-pyrazol-4-yl)-1H-pyrrolo[2,3-b]pyridin-3-yl]-pyrimidin-2-ylamin 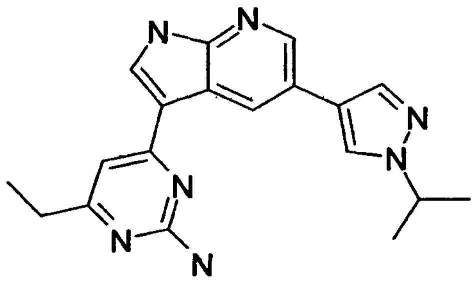 | ESI 348 |
| "A64" | 4-Ethyl-6-[5-(1,3,5-trimethyl-1H-pyrazol-4-yl)-1H-pyrrolo[2,3-b]pyridin-3-yl]-pyrimidin-2-ylamin 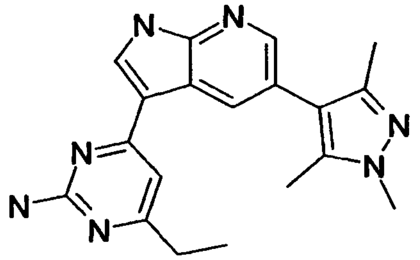 | ESI 348 |

(fortgesetzt)

| Verbindung Nr. | Name und/oder Struktur | Analytische Daten |
|---|---|---|
| "A65" | 4-[3-(2-Amino-6-ethyl-pyrimidin-4-yl)-1H-pyrrolo[2,3-b]pyridin-5-yl]-benzamid 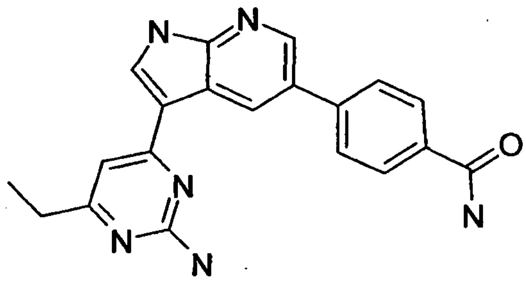 | ESI 359 |
| "A66" | 4-Ethyl-6-[5-(1-isobutyl-1H-pyrazol-4-yl)-1H-pyrrolo[2,3-b]pyridin-3-yl]-pyrimidin-2-ylamin 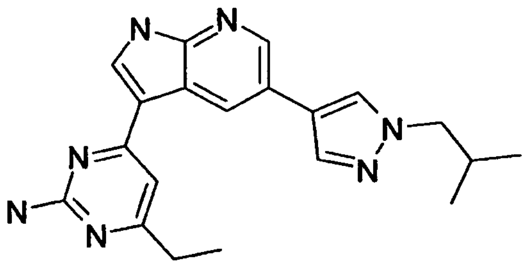 | ESI 362 |
| "A67" | 4-(5-Bromo-1H-pyrrolo[2,3-b]pyridin-3-yl)-6-phenyl-pyrimidin-2-ylamin 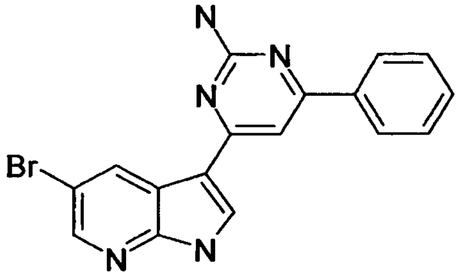 | ESI 367 |

Tabelle 1 Inhibierung auf die Proliferation/ Vitalität von Tumorzellen/ Inhibierung von PDK1 $IC_{50}$ von erfindungsgemäßen Verbindungen

| Verbindung Nr. | $IC_{50}$ Zellen A2780 (Eierstock) |
|---|---|
| "A1" | A |
| "A2" | A |
| "A3" | A |
| "A5" | A |
| "A6" | B |
| "A8" | B |
| Verbindung Nr. | $IC_{50}$ PDK1 |
| "A16" | A |
| "A32" | A |
| "A42" | A |

(fortgesetzt)

| Verbindung Nr. | $IC_{50}$ PDK1 |
|---|---|
| "A43" | A |
| "A45" | A |
| "A48" | A |
| "A49" | A |
| "A56" | A |
| "A60" | A |
| "A66" | A |
| $IC_{50}$: 10 nM - 1 µM = A<br>1 µM-10 µM = B<br>> 10 µM = C | |

Die nachfolgenden Beispiele betreffen Arzneimittel:

**Beispiel A: Injektionsgläser**

Eine Lösung von 100 g eines Wirkstoffes der Formel I und 5 g Dinatriumhydrogenphosphat wird in 3 l zweifach destilliertem Wasser mit 2 N Salzsaure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

**Beispiel B: Suppositorien**

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

**Beispiel C: Lösung**

Man bereitet eine Lösung aus 1 g eines Wirkstoffes der Formel I, 9,38 g $NaH_2PO_4 \cdot 2 H_2O$, 28,48 g $Na_2HPO_4 \cdot 12 H_2O$ und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

**Beispiel D: Salbe**

Man mischt 500 mg eines Wirkstoffes der Formel I mit 99,5 g Vaseline unter aseptischen Bedingungen.

**Beispiel E: Tabletten**

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

**Beispiel F: Dragees**

Analog Beispiel E werden Tabletten gepreßt, die anschließend in üblicher Weise mit einem Überzug aus Saccharose, Kartoffelstärke, Talk, Tragant und Farbstoff überzogen werden.

**Beispiel G: Kapseln**

2 kg Wirkstoff der Formel I werden in üblicher Weise in Hartgelatinekapseln gefüllt, so daß jede Kapsel 20 mg des Wirkstoffs enthält.

**Beispiel H: Ampullen**

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I

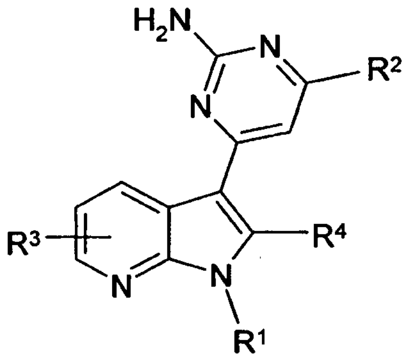

I

worin

$R^1$ H, A oder $-[C(R^5)_2]_nAr$,
$R^2$ A, $-[C(R^5)_2]_nAr$, $-[C(R^5)_2]_nHet$, $-[C(R^5)2]_nCycloalkyl$, $-[C(R^5)_2]nOR^5$, $-[C(R^5)_2]nN(R^5)_2$ oder $-[C(R^5)_2]_nCOOR^5$,
$R^3$ H, A, Hal, CN, $-[C(R^5)_2]_nAr$, $-[C(R^5)_2]_nHet$ oder $-[C(R^5)_2]_nCycloalkyl$ oder $-[C(R^5)_2]_nN(R^5)_2$,
$R^4$ H, A, $-[C(R^5)_2]_nAr$, $-[C(R^5)_2]_nHet$ oder $-[C(R^5)_2]_nCycloalkyl$,
$R^5$ H oder Alkyl mit 1-6 C-Atomen,
A unverzweigtes oder verzweigtes Alkyl mit 1-10 C-Atomen, worin eine oder zwei $CH_2$-Gruppen durch O-oder S-Atome und/oder durch -CH=CH-Gruppen und/oder auch 1-7 H-Atome durch F ersetzt sein können,
Cycloalkyl Cyclisches Alkyl mit 3-7 C-Atomen, das zusätzlich durch Alkyl mit 1-6 C-Atomen substituiert sein kann,
Hal F, Cl, Br oder I,
Ar unsubstituiertes oder ein-, zwei- oder dreifach durch Hal, A, $OR^5$, $N(R^5)_2$, $NO_2$, CN, $COOR^5$, $CON(R^5)_2$, $NR^5COA$, $NR^5SO_2A$, $COR^5$, $SO_2N(R^5)_2$ und/oder $S(O)_pA$ substituiertes Phenyl,
Het einen unsubstituierten, ein-, zwei- oder dreifach durch Hal, A, $OR^5$, $N(R^5)_2$, $NO_2$, CN, $COOR^5$, $CON(R^5)_2$, $NR^5COA$, $NR^5SO_2A$, $COR^5$, $SO_2NR^5$, $S(O)_pA$ und/oder =O (Carbonylsauerstoff) substituierten ein- oder zweikernigen gesättigten, ungesättigten oder aromatischen Heterocyclus mit 1 bis 4 N-, und/oder O-und/oder S-Atomen,
n 0, 1, 2, 3 oder 4,
p 0, 1 oder 2

bedeuten,
sowie ihre pharmazeutisch verwendbaren Solvate, Salze, Tautomere und Stereoisomere, einschließlich deren Mischungen in allen Verhältnissen.

2. Verbindungen nach Anspruch 1, worin

$R^1$ H, oder $-[C(R^5)_2]_nAr$,

bedeutet,
sowie ihre pharmazeutisch verwendbaren Solvate, Salze, Tautomere und Stereoisomere, einschließlich deren Mischungen in allen Verhältnissen.

3. Verbindungen nach Anspruch 1 oder 2, worin

$R^3$ H, Br, Cl, CN, Ar oder Het bedeutet,

sowie ihre pharmazeutisch verwendbaren Solvate, Salze, Tautomere und Stereoisomere, einschließlich deren Mischungen in allen Verhältnissen.

**4.** Verbindungen nach Anspruch 1, 2 oder 3, worin

$R^4$ H oder A bedeutet,

sowie ihre pharmazeutisch verwendbaren Solvate, Salze, Tautomere und Stereoisomere, einschließlich deren Mischungen in allen Verhältnissen.

**5.** Verbindungen nach einem oder mehreren der Ansprüche 1-4, worin

$R^5$ H oder A bedeutet,

sowie ihre pharmazeutisch verwendbaren Solvate, Salze, Tautomere und Stereoisomere, einschließlich deren Mischungen in allen Verhältnissen.

**6.** Verbindungen nach einem oder mehreren der Ansprüche 1-5, worin

A unverzweigtes oder verzweigtes Alkyl mit 1-8 C-Atomen, worin eine oder zwei $CH_2$-Gruppen durch Sauerstoff und/oder auch 1-7 H-Atome durch F ersetzt sein können,

bedeutet,
sowie ihre pharmazeutisch verwendbaren Solvate, Salze, Tautomere und Stereoisomere, einschließlich deren Mischungen in allen Verhältnissen.

**7.** Verbindungen nach einem oder mehreren der Ansprüche 1-6, worin

A unverzweigtes oder verzweigtes Alkyl mit 1-6 C-Atomen, worin eine oder zwei $CH_2$-Gruppen durch Sauerstoff und/oder auch 1-7 H-Atome durch F ersetzt sein können,

bedeutet,
sowie ihre pharmazeutisch verwendbaren Solvate, Salze, Tautomere und Stereoisomere, einschließlich deren Mischungen in allen Verhältnissen.

**8.** Verbindungen nach einem oder mehreren der Ansprüche 1-7, worin

Ar unsubstituiertes oder ein-, zwei- oder dreifach durch A, $NO_2$, $N(R^5)_2$, CN und/oder $COOR^5$ substituiertes Phenyl

bedeutet,
sowie ihre pharmazeutisch verwendbaren Solvate, Salze, Tautomere und Stereoisomere, einschließlich deren Mischungen in allen Verhältnissen.

**9.** Verbindungen nach einem oder mehreren der Ansprüche 1-8, worin

Het einen unsubstituierten oder ein-, zwei- oder dreifach durch A, OH, OA, Hal, $CON(R^5)_2$, $N(R^5)_2$ und/oder =O (Carbonylsauerstoff) substituierten ein- oder zweikernigen gesättigten, ungesättigten oder aromatischen Heterocyclus mit 1 bis 3 N- und/oder O-Atomen,

bedeutet,
sowie ihre pharmazeutisch verwendbaren Solvate, Salze, Tautomere und Stereoisomere, einschließlich deren Mischungen in allen Verhältnissen.

**10.** Verbindungen nach einem oder mehreren der Ansprüche 1-9, worin

Het unsubstituiertes oder ein-, zwei- oder dreifach durch A, OH, OA, Hal, $CON(R^5)_2$, $N(R^5)_2$ und/oder =O substituiertes Dihydro-pyrrolyl, Pyrrolidinyl, Tetrahydroimidazolyl, Dihydro-pyrazolyl, Tetrahydro-pyrazolyl, Dihydro-pyridyl, Tetrahydro-pyridyl, Piperidinyl, Morpholinyl, Hexahydro-pyridazinyl, Hexahydropyrimidinyl, Piperazinyl, 1,2,3,4-Tetrahydro-chinolyl, 1,2,3,4-Tetrahydro-isochinolyl, 3,4-Dihydro-benzo[1,4]oxazinyl, 2,3-Dihydro-isoindolyl, 2,3-Dihydro-indolyl, Oxazolidinyl, Isoxazolidinyl, Oxadiazolidinyl, Thiazolidinyl, Furyl, Thienyl, Pyrrolyl, Imidazolyl, Pyrazolyl, Oxazolyl, Isoxazolyl, Thiazolyl, Pyridyl, Pyrimidinyl, Triazolyl, Tetrazolyl, Thiadiazol, Pyridazinyl, Pyrazinyl, Indolyl, Isoindolyl, Benzimidazolyl, Indazolyl, Chinolyl oder 1,3-Benzodioxolyl,

bedeutet,
sowie ihre pharmazeutisch verwendbaren Solvate, Salze, Tautomere und Stereoisomere, einschließlich deren Mischungen in allen Verhältnissen.

**11.** Verbindungen nach einem oder mehreren der Ansprüche 1-10,
worin

Het unsubstituiertes oder ein- oder zwei-, oder dreifach durch A, $CON(R^5)_2$, $N(R^5)_2$ oder F und/oder =O substituiertes Dihydro-pyrrolyl, Pyrrolidinyl, Tetrahydroimidazolyl, Dihydro-pyrazolyl, Tetrahydro-pyrazolyl, Dihydropyridyl, Tetrahydro-pyridyl, Piperidinyl, Morpholinyl, Hexahydro-pyridazinyl, Hexahydro-pyrimidinyl, Piperazinyl, 1,2,3,4-Tetrahydro-chinolyl, 1,2,3,4-Tetrahydro-isochinolyl, 3,4-Dihydro-benzo[1,4]oxazinyl, 2,3-Dihydro-isoindolyl, 2,3-Dihydro-indolyl, Pyrazolyl, Pyridyl, Furyl oder Isooxazol,

bedeutet,
sowie ihre pharmazeutisch verwendbaren Solvate, Salze, Tautomere und Stereoisomere, einschließlich deren Mischungen in allen Verhältnissen.

**12.** Verbindungen nach einem oder mehreren der Ansprüche 1-11, worin

$R^1$ H oder $-[C(R^5)_2]_nAr$,
$R^2$ A, $-[C(R^5)_2]_nAr$, $-[C(R^5)_2]_nHet$, $-[C(R^5)_2]_n$Cycloalkyl, $-[C(R^5)_2]_nOR^5$, $-[C(R^5)_2]_nN(R^5)_2$ oder $-[C(R^5)_2]_nCOOR^5$,
$R^3$ H, Br, Cl, CN, Ar oder Het,
$R^4$ H oder A,
$R^5$ H oder A,
A unverzweigtes oder verzweigtes Alkyl mit 1-6 C-Atomen, worin eine oder zwei $CH_2$-Gruppen durch Sauerstoff und/oder auch 1-7 H-Atome durch F ersetzt sein können,
Ar unsubstituiertes oder ein-, zwei- oder dreifach durch A, $NO_2$, $N(R^5)_2$, CN und/oder $COOR^5$ substituiertes Phenyl,
Het unsubstituiertes oder ein-, zwei- oder dreifach durch A, $CON(R^5)_2$, $N(R^5)_2$ oder F und/oder =O substituiertes Dihydro-pyrrolyl, Pyrrolidinyl, Tetrahydro-imidazolyl, Dihydro-pyrazolyl, Tetrahydro-pyrazolyl, Dihydro-pyridyl, Tetrahydro-pyridyl, Piperidinyl, Morpholinyl, Hexahydropyridazinyl, Hexahydro-pyrimidinyl, Piperazinyl, 1,2,3,4-Tetrahydro-chinolyl, 1,2,3,4-Tetrahydro-isochinolyl, 3,4-Dihydro-benzo[1,4]oxazinyl, 2,3-Dihydro-isoindolyl, 2,3-Dihydro-indolyl, Pyrazolyl, Pyridyl, Furyl oder Isooxazol,
n 0, 1, 2, 3 oder 4,

bedeuten,
sowie ihre pharmazeutisch verwendbaren Solvate, Salze, Tautomere und Stereoisomere, einschließlich deren Mischungen in allen Verhältnissen.

**13.** Verbindungen nach Anspruch 1, ausgewählt aus der Gruppe

| Verbindung Nr. | Name und/oder Struktur |
|---|---|
| "A1" | 4-Butyl-6-(1H-pyrrolo[2,3-b]pyridin-3-yl)-pyrimidin-2-ylamin |
| "A2" | 4-Phenyl-6-(1H-pyrrolo[2,3-b]pyridin-3-yl)-pyrimidin-2-ylamin |
| "A3" | 4-Propyl-6-(1H-pyrrolo[2,3-b]pyridin-3-yl)-pyrimidin-2-ylamin |
| "A4" | 4-Methoxymethyl-6-(1H-pyrrolo[2,3-b]pyridin-3-yl)-pyrimidin-2-ylamin |

(fortgesetzt)

| Verbindung Nr. | Name und/oder Struktur |
|---|---|
| | 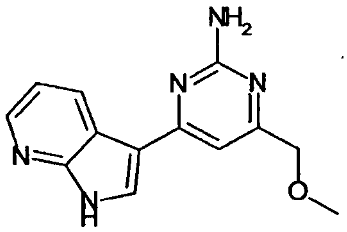 |
| "A5" | 4-Ethyl-6-(1H-pyrrolo[2,3-b]pyridin-3-yl)-pyrimidin-2-ylamin |
| "A6" | 4-Pentyl-6-(1H-pyrrolo[2,3-b]pyridin-3-yl)-pyrimidin-2-ylamin |
| "A7" | 4-(1-Benzyl-1H-pyrrolo[2,3-b]pyridin-3-yl)-6-butyl-pyrimidin-2-ylamin |
| "A7a" | 4-[1-(4-Methoxybenzyl)-1H-pyrrolo[2,3-b]pyridin-3-yl]-6-methoxymethyl-pyrimidin-2-ylamin |
| "A8" | 4-(4-Propyl-cyclohexyl)-6-(1H-pyrrolo[2,3-b]pyridin-3-yl)-pyrimidin-2-ylamin 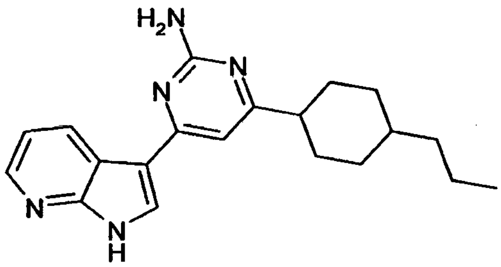 |
| "A9" | 4-(4-Propyl-phenyl)-6-(1H-pyrrolo[2,3-b]pyridin-3-yl)-pyrimidin-2-ylamin |
| "A10" | 4-Methyl-6-(1H-pyrrolo[2,3-b]pyridin-3-yl)-pyrimidin-2-ylamin |
| "A11" | 4-Hydroxymethyl-6-(1H-pyrrolo[2,3-b]pyridin-3-yl)-pyrimidin-2-ylamin |
| "A12" | 2-[2-Amino-6-(1H-pyrrolo[2,3-b]pyridin-3-yl)-pyrimidin-4-ylmethyl]isoindol-1,3-dion |
| "A13" | 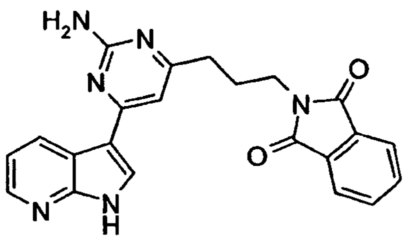 |
| "A14" | 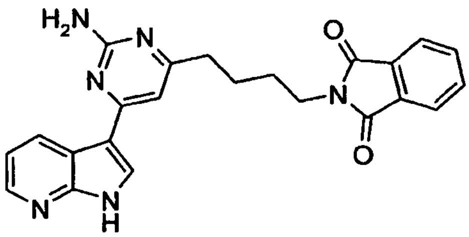 |
| "A15" | 4-Aminomethyl-6-(pyrrolo[2,3-b]pyridin-3-yl)-pyrimidin-2-ylamin |

(fortgesetzt)

| Verbindung Nr. | Name und/oder Struktur |
|---|---|
| "A16" | 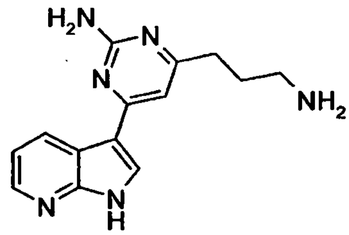 |
| "A17" | 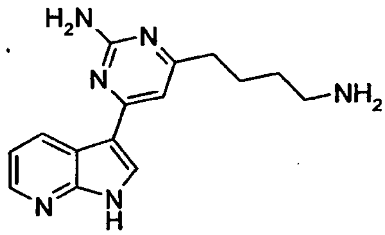 |
| "A18" | 4-(N, N-Dimethylaminomethyl)-6-(pyrrölo[2,3-b]pyridin-3-yl)-pyrimidin-2-ylamin |
| "A19" | 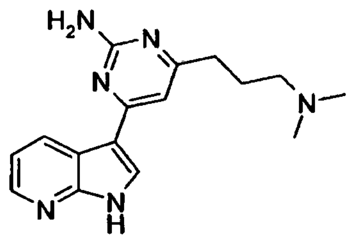 |
| "A20" | 4-(Pyrrolidin-1-yl-propyl)-6-(1H-pyrrolo[2,3-b]pyridin-3-yl)-pyrimidin-2-ylamin |
| "A21" | 4-(Piperidin-4-yl-methyl)-6-(1H-pyrrolo[2,3-b]pyridin-3-yl)-pyrimidin-2-ylamin |
| "A22" | 4-(1-Methyl-piperidin-4-yl-methyl)-6-(1H-pyrrolo[2,3-b]pyridin-3-yl)-pyrimidin-2-ylamin |
| "A23" | 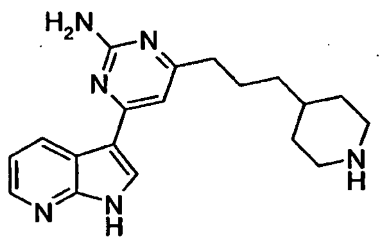 |
| "A24" | 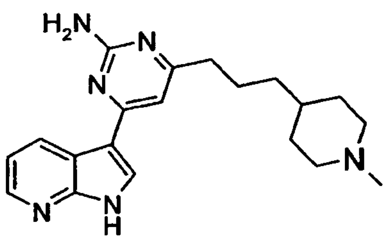 |
| "A25" | 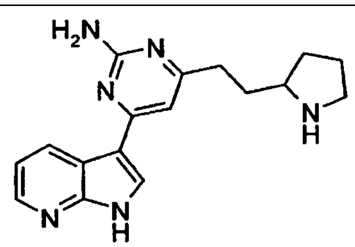 |

(fortgesetzt)

| Verbindung Nr. | Name und/oder Struktur |
|---|---|
| "A26" | 3-[2-Amino-6-(1H-pyrrolo[2,3-b]pyridin-3-yl)-pyrimidin-4-yl]-propan-1-ol |
| "A27" | 4-Methoxymethyl-6-(1H-pyrrolo[2,3-b]pyridin-3-yl)-pyrimidin-2-ylamin |
| "A28" | 3-[2-Amino-6-(1H-pyrrolo[2,3-b]pyridin-3-yl)-pyrimidin-4-yl]-propionsäure |
| "A29" | 4-[2-Amino-6-(1H-pyrrolo[2,3-b]pyridin-3-yl)-pyrimidin-4-yl]-butan-1-ol |
| "A30" | 4-[2-Amino-6-(1H-pyrrolo[2,3-b]pyridin-3-yl)-pyrimidin-4-yl]-buttersäure |
| "A31" | 4-Methyl-6-(1H-pyrrolo[2,3-b]pyridin-3-yl)-pyrimidin-2-ylamin |
| "A32" | 4-(5-Bromo-1H-pyrrolo[2,3-b]pyridin-3-yl)-6-ethyl-pyrimidin-2-ylamin |
| "A33" | 4-(3-Nitro-phenyl)-6-(1H-pyrrolo[2,3-b]pyridin-3-yl)-pyrimidin-2-ylamin |
| "A34" | 4-(3-Amino-phenyl)-6-(1H-pyrrolo[2,3-b]pyridin-3-yl)-pyrimidin-2-ylamin |
| "A35" | 4-Ethyl-6-(2-methyl-1H-pyrrolo[2,3-b]pyridin-3-yl)-pyrimidin-2-ylamin |
| "A36" | 4-(4-Chloro-1H-pyrrolo[2,3-b]pyridin-3-yl)-6-ethyl-pyrimidin-2-ylamin |
| "A37" | 3-(2-Amino-6-ethyl-pyrimidin-4-yl)-1H-pyrrolo[2,3-b]pyridine-5-carbonitril |
| "A38" | [2-Amino-6-(1-benzyl-1H-pyrrolo[2,3-b]pyridin-3-yl)-pyrimidin-4-yl]-methanol |
| "A39" | 4-(3-Piperazin-1-yl-propyl)-6-(1H-pyrrolo[2,3-b]pyridin-3-yl)-pyrimidin-2-ylamin |
| "A40" | 4-(3-Morpholin-4-yl-propyl)-6-(1H-pyrrolo[2,3-b]pyridin-3-yl)-pyrimidin-2-ylamin |
| "A41" | 4-[3-(4-Methyl-piperazin-1-yl)-propyl]-6-(1H-pyrrolo[2,3-b]pyridin-3-yl)-pyrimidin-2-ylamin |
| "A42" | 4-Ethyl-6-{5-[1-(1-methyl-piperidin-4-yl)-1H-pyrazol-4-yl]-1H-pyrrolo[2,3-b]pyridin-3-yl} -pyrimidin-2-ylamin |
| "A43" | 4-(3-Amino-propyl)-6-(5-furan-2-yl-1H-pyrrolo[2,3-b]pyridin-3-yl)-pyrimidin-2-ylamin |
| "A44" | 4-(3-Amino-propyl)-6-[5-(1-methyl-1H-pyrazol-4-yl)-1H-pyrrolo[2,3-b]pyridin-3-yl]-pyrimidin-2-ylamin |
| "A45" | 4-Ethyl-6-[5-(1-methyl-1H-pyrazol-4-yl)-1H-pyrrolo[2,3-b]pyridin-3-yl]-pyrimidin-2-ylamin |
| "A46" | 4-Ethyl-6-(5-pyridin-3-yl-1H-pyrrolo[2,3-b]pyridin-3-yl)-pyrimidin-2-ylamin |
| "A47" | 4-Ethyl-6-(5-pyridin-4-yl-1H-pyrrolo[2,3-b]pyridin-3-yl)-pyrimidin-2-ylamin |
| "A48" | 4-[5-(6-Amino-pyridin-3-yl)-1H-pyrrolo[2,3-b]pyridin-3-yl]-6-ethyl-pyrimidin-2-ylamin |
| "A49" | 4-Ethyl-6-(5-furan-2-yl-1H-pyrrolo[2,3-b]pyridin-3-yl)-pyrimidin-2-ylamin |
| "A50" | 3-Amino-5-[3-(2-amino-6-ethyl-pyrimidin-4-yl)-1H-pyrrolo[2,3-b]pyridin-5-yl]-benzoesäure |
| "A51" | 4-[5-(3,5-Dimethyl-isoxazol-4-yl)-1H-pyrrolo[2,3-b]pyridin-3-yl]-6-ethyl-pyrimidin-2-ylamin |
| "A52" | 4-Ethyl-6-(5-phenyl-1H-pyrrolo[2,3-b]pyridin-3-yl)-pyrimidin-2-ylamin |
| "A53" | 4-[5-(6-Dimethylamino-pyridin-3-yl)-1H-pyrrolo[2,3-b]pyridin-3-yl]-6-ethyl-pyrimidin-2-ylamin |
| "A54" | 3-[3-(2-Amino-6-ethyl-pyrimidin-4-yl)-1H-pyrrolo[2,3-b]pyridin-5-yl]-benzonitril |
| "A55" | 4-Ethyl-6-{5-[1-(3-methoxy-propyl)-1H-pyrazol-4-yl]-1H-pyrrolo[2,3-b]pyridin-3-yl}-pyrimidin-2-ylamin |
| "A56" | 4-Ethyl-6-{5-[1-(2-methoxy-ethyl)-1H-pyrazol-4-yl]-1H-pyrrolo[2,3-b]pyridin-3-yl}-pyrimidin-2-ylamin |
| "A57" | 4-Ethyl-6-[5-(6-fluoro-4-methyl-pyridin-3-yl)-1H-pyrrolo[2,3-b]pyridin-3-yl]-pyrimidin-2-ylamin |
| "A58" | 5-[3-(2-Amino-6-ethyl-pyrimidin-4-yl)-1H-pyrrolo[2,3-b]pyridin-5-yl]-pyridine-2-carboxylic acid methylamid |

(fortgesetzt)

| Verbindung Nr. | Name und/oder Struktur |
|---|---|
| "A59" | 4-Ethyl-6-{5-[1-(3-methyl-butyl)-1H-pyrazol-4-yl]-1H-pyrrolo[2,3-b]pyridin-3-yl}-pyrimidin-2-ylamin |
| "A60" | 4-Ethyl-6-[5-(1H-pyrazol-4-yl)-1H-pyrrolo[2,3-b]pyridin-3-yl]-pyrimidin-2-ylamin |
| "A61" | 4-Ethyl-6-[5-(2H-pyrazol-3-yl)-1H-pyrrolo[2,3-b]pyridin-3-yl]-pyrimidin-2-ylamin |
| "A62" | 4-Ethyl-6-[5-(2-methyl-2H-pyrazol-3-yl)-1H-pyrrolo[2,3-b]pyridin-3-yl]-pyrimidin-2-ylamin |
| "A63" | 4-Ethyl-6-[5-(1-isopropyl-1H-pyrazol-4-yl)-1H-pyrrolo[2,3-b]pyridin-3-yl]-pyrimidin-2-ylamin |
| "A64" | 4-Ethyl-6-[5-(1,3,5-trimethyl-1H-pyrazol-4-yl)-1H-pyrrolo[2,3-b]pyridin-3-yl]-pyrimidin-2-ylamin |
| "A65" | 4-[3-(2-Amino-6-ethyl-pyrimidin-4-yl)-1H-pyrrolo[2,3-b]pyridin-5-yl]-benzamid |
| "A66" | 4-Ethyl-6-[5-(1-isobutyl-1H-pyrazol-4-yl)-1H-pyrrolo[2,3-b]pyridin-3-yl]-pyrimidin-2-ylamin |
| "A67" | 4-(5-Bromo-1H-pyrrolo[2,3-b]pyridin-3-yl)-6-phenyl-pyrimidin-2-ylamin |

sowie ihre pharmazeutisch verwendbaren Solvate, Salze, Tautomere und Stereoisomere, einschließlich deren Mischungen in allen Verhältnissen.

**14.** Verfahren zur Herstellung von Verbindungen der Formel I nach den Ansprüchen 1-13 sowie ihrer pharmazeutisch verwendbaren Salze, Solvate, Tautomeren und Stereoisomeren, **dadurch gekennzeichnet, daß** man

a) eine Verbindung der Formel II

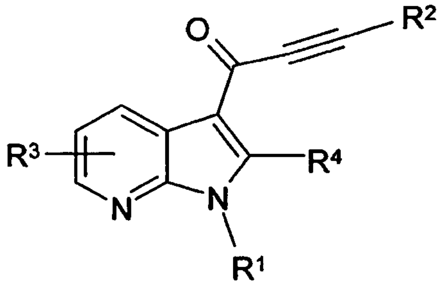

II

,

worin $R^1$ eine Indolschutzgruppe bedeutet,
$R^2$, $R^3$ und $R^4$ die in Anspruch 1 angegebenen Bedeutungen haben,
mit Guanidin umsetzt,
und gleichzeitig oder anschließend die Indolschutzgruppe abspaltet,
oder
b) in einer Verbindung der Formel I einen Rest $R^2$ in einen anderen $R^2$ umwandelt,
indem man

i) eine Aminoschutzgruppe abspaltet,
ii) ein Imid zu einem Amin spaltet,
iii) eine Alkylierung durchführt,

und/oder
eine Base oder Säure der Formel I in eines ihrer Salze umwandelt.

**15.** Arzneimittel, enthaltend mindestens eine Verbindung der Formel I nach Anspruch 1-13 und/oder ihre pharmazeutisch verwendbaren Salze, Solvate, Tautomeren und Stereoisomeren, einschließlich deren Mischungen in allen Verhältnissen, sowie gegebenenfalls Träger- und/oder Hilfsstoffe.

**16.** Verwendung von Verbindungen nach Anspruch 1-13 sowie ihrer pharmazeutisch verwendbaren Salze, Solvate,

Tautomeren und Stereoisomeren, einschließlich deren Mischungen in allen Verhältnissen, zur Herstellung eines Arzneimittels zur Behandlung von Tumoren, Tumorwachstum, Tumormetastasen und/oder AIDS.

**17.** Verwendung nach Anspruch 16, wobei der Tumor aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopfs und/oder der Lunge stammt.

**18.** Verwendung nach Anspruch 16, wobei der Tumor aus der Gruppe Monozytenleukämie, Lungenadenokarzinom, kleinzellige Lungenkarzinome, Bauchspeicheldrüsenkrebs, Kolonkarzinom, Glioblastome und/oder Brustkarzinom stammt.

**19.** Verwendung nach Anspruch 16, wobei es sich um einen Tumor des Blut- und Immunsystems handelt.

**20.** Verwendung nach Anspruch 16, wobei der Tumor aus der Gruppe der akuten myelotischen Leukämie, der chronischen myelotischen Leukämie, akuten lymphatischen Leukämie und/oder chronischen lymphatischen Leukämie stammt.

**21.** Verwendung von Verbindungen der Formel I gemäß Anspruch 1-13 und/oder ihrer physiologisch unbedenklichen Salze und Solvate zur Herstellung eines Arzneimittels zur Behandlung von Tumoren, wobei eine therapeutisch wirksame Menge einer Verbindung der Formel I in Kombination mit einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

**22.** Verwendung von Verbindungen der Formel I gemäß Anspruch 1-13 und/oder ihrer physiologisch unbedenklichen Salze und Solvate zur Herstellung eines Arzneimittels zur Behandlung von Tumoren wobei eine therapeutisch wirksame Menge einer Verbindung der Formel I in Kombination mit Radiotherapie und einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

## Claims

**1.** Compounds of the formula I

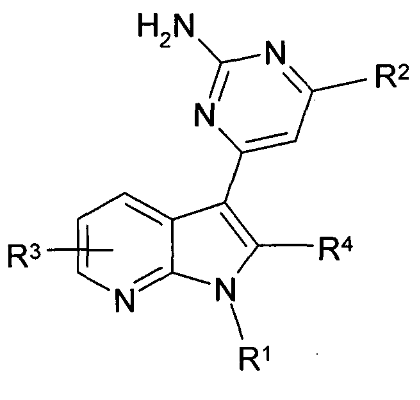

I

in which

$R^1$ denotes H, A or $-[C(R^5)_2]_nAr$,
$R^2$ denotes A, $-[C(R^5)_2]_nAr$, $-[C(R^5)_2]_nHet$, $-[C(R^5)_2]_n$cycloalkyl, $-[C(R^5)_2]_nOR^5$, $-[C(R^5)_2]_nN(R^5)_2$ or $-[C(R^5)_2]_nCOOR^5$,
$R^3$ denotes H, A, Hal, CN, $-[C(R^5)_2]_nAr$, $-[C(R^5)_2]_nHet$ or $-[C(R^5)_2]_n$cycloalkyl or $-[C(R^5)_2]_nN(R^5)_2$,
$R^4$ denotes H, A, $-[C(R^5)_2]_nAr$, $-[C(R^5)_2]_nHet$ or $-[C(R^5)_2]_n$-cycloalkyl,
$R^5$ denotes H or alkyl having 1-6 C atoms,

A denotes unbranched or branched alkyl having 1-10 C atoms, in which one or two $CH_2$ groups may be replaced by O or S atoms and/or by -CH=CH- groups and/or in addition 1-7 H atoms may be replaced by F,
cycloalkyl denotes cyclic alkyl having 3-7 C atoms, which may additionally be substituted by alkyl having 1-6 C atoms,
Hal denotes F, Cl, Br or I,
Ar denotes phenyl which is unsubstituted or mono-, di- or trisubstituted by Hal, A, $OR^5$, $N(R^5)_2$, $NO_2$, CN, $COOR^5$, $CON(R^5)_2$, $NR^5COA$, $NR^5SO_2A$, $COR^5$, $SO_2N(R^5)_2$ and/or $S(O)_pA$,
Het denotes a mono- or bicyclic saturated, unsaturated or aromatic heterocycle having 1 to 4 N and/or O and/or S atoms which is unsubstituted, mono-, di- or trisubstituted by Hal, A, $OR^5$, $N(R^5)_2$, $NO_2$, CN, $COOR^5$, $CON(R^5)_2$, $NR^5COA$, $NR^5SO_2A$, $COR^5$, $SO_2NR^5$, $S(O)_pA$ and/or =O (carbonyl oxygen),
n denotes 0, 1, 2, 3 or 4,
p denotes 0, 1 or 2,

and pharmaceutically usable solvates, salts, tautomers and stereoisomers thereof, including mixtures thereof in all ratios.

**2.** Compounds according to Claim 1 in which

$R^1$ denotes H or $-[C(R^5)_2]_nAr$,

and pharmaceutically usable solvates, salts, tautomers and stereoisomers thereof, including mixtures thereof in all ratios.

**3.** Compounds according to Claim 1 or 2 in which

$R^3$ denotes H, Br, Cl, CN, Ar or Het,

and pharmaceutically usable solvates, salts, tautomers and stereoisomers thereof, including mixtures thereof in all ratios.

**4.** Compounds according to Claim 1, 2 or 3 in which

$R^4$ denotes H or A,

and pharmaceutically usable solvates, salts, tautomers and stereoisomers thereof, including mixtures thereof in all ratios.

**5.** Compounds according to one or more of Claims 1-4 in which

$R^5$ denotes H or A,

and pharmaceutically usable solvates, salts, tautomers and stereoisomers thereof, including mixtures thereof in all ratios.

**6.** Compounds according to one or more of Claims 1-5 in which

A denotes unbranched or branched alkyl having 1-8 C atoms, in which one or two $CH_2$ groups may be replaced by oxygen and/or in addition 1-7 H atoms may be replaced by F,

and pharmaceutically usable solvates, salts, tautomers and stereoisomers thereof, including mixtures thereof in all ratios.

**7.** Compounds according to one or more of Claims 1-6 in which

A denotes unbranched or branched alkyl having 1-6 C atoms, in which one or two $CH_2$ groups may be replaced by oxygen and/or in addition 1-7 H atoms may be replaced by F,

and pharmaceutically usable solvates, salts, tautomers and stereoisomers thereof, including mixtures thereof in all

ratios.

**8.** Compounds according to one or more of Claims 1-7 in which

Ar denotes phenyl which is unsubstituted or mono-, di- or trisubstituted by A, $NO_2$, $N(R^5)_2$, CN and/or $COOR^5$,

and pharmaceutically usable solvates, salts, tautomers and stereoisomers thereof, including mixtures thereof in all ratios.

**9.** Compounds according to one or more of Claims 1-8 in which

Het denotes a mono- or bicyclic saturated, unsaturated or aromatic heterocycle having 1 to 3 N and/or O atoms which is unsubstituted or mono-, di- or trisubstituted by A, OH, OA, Hal, $CON(R^5)_2$, $N(R^5)_2$ and/or =O (carbonyl oxygen),

and pharmaceutically usable solvates, salts, tautomers and stereoisomers thereof, including mixtures thereof in all ratios.

**10.** Compounds according to one or more of Claims 1-9 in which

Het denotes dihydropyrrolyl, pyrrolidinyl, tetrahydroimidazolyl, dihydropyrazolyl, tetrahydropyrazolyl, dihydropyridyl, tetrahydropyridyl, piperidinyl, morpholinyl, hexahydropyridazinyl, hexahydropyrimidinyl, piperazinyl, 1,2,3,4-tetrahydroquinolyl, 1,2,3,4-tetrahydroisoquinolyl, 3,4-dihydrobenzo-1,4-oxazinyl, 2,3-dihydroisoindolyl, 2,3-dihydroindolyl, oxazolidinyl, isoxazolidinyl, oxadiazolidinyl, thiazolidinyl, furyl, thienyl, pyrrolyl, imidazolyl, pyrazolyl, oxazolyl, isoxazolyl, thiazolyl, pyridyl, pyrimidinyl, triazolyl, tetrazolyl, thiadiazole, pyridazinyl, pyrazinyl, indolyl, isoindolyl, benzimidazolyl, indazolyl, quinolyl or 1,3-benzodioxolyl, each of which is unsubstituted or mono-, di- or trisubstituted by A, OH, OA, Hal, $CON(R^5)_2$, $N(R^5)_2$ and/or =O,

and pharmaceutically usable solvates, salts, tautomers and stereoisomers thereof, including mixtures thereof in all ratios.

**11.** Compounds according to one or more of Claims 1-10 in which

Het denotes dihydropyrrolyl, pyrrolidinyl, tetrahydroimidazolyl, dihydropyrazolyl, tetrahydropyrazolyl, dihydropyridyl, tetrahydropyridyl, piperidinyl, morpholinyl, hexahydropyridazinyl, hexahydropyrimidinyl, piperazinyl, 1,2,3,4-tetrahydroquinolyl, 1,2,3,4-tetrahydroisoquinolyl, 3,4-dihydrobenzo-1,4-oxazinyl, 2,3-dihydroisoindolyl, 2,3-dihydroindolyl, pyrazolyl, pyridyl, furyl or isoxazole, each of which is unsubstituted or mono-, di- or trisubstituted by A, $CON(R^5)_2$, $N(R^5)_2$ or F and/or =O,

and pharmaceutically usable solvates, salts, tautomers and stereoisomers thereof, including mixtures thereof in all ratios.

**12.** Compounds according to one or more of Claims 1-11 in which

$R^1$ denotes H or $-[C(R^5)_2]_nAr$,
$R^2$ denotes A, $-[C(R_5)_2]_nAr$, $-[C(R^5)_2]_nHet$, $-[C(R^5)_2]_n$cycloalkyl, $-[C(R^5)_2]_nOR^5$, $-[C(R^5)_2]_nN(R^5)_2$ or $-[C(R^5)_2]_n-COOR^5$,
$R^3$ denotes H, Br, Cl, CN, Ar or Het,
$R^4$ denotes H or A,
$R^5$ denotes H or A,
A denotes unbranched or branched alkyl having 1-6 C atoms, in which one or two $CH_2$ groups may be replaced by oxygen and/or in addition 1-7 H atoms may be replaced by F,
Ar denotes phenyl which is unsubstituted or mono-, di- or trisubstituted by A, $NO_2$, $N(R^5)_2$, CN and/or $COOR^5$,
Het denotes dihydropyrrolyl, pyrrolidinyl, tetrahydroimidazolyl, dihydropyrazolyl, tetrahydropyrazolyl, dihydropyridyl, tetrahydropyridyl, piperidinyl, morpholinyl, hexahydropyridazinyl, hexahydropyrimidinyl, piperazinyl, 1,2,3,4-tetrahydroquinolyl, 1,2,3,4-tetrahydroisoquinolyl, 3,4-dihydrobenzo-1,4-oxazinyl, 2,3-dihydroisoindolyl, 2,3-dihydroindolyl, pyrazolyl, pyridyl, furyl or isoxazole, each of which is unsubstituted or mono-, di- or trisubstituted by A, $CON(R^5)_2$, $N(R^5)_2$ or F and/or =O,

n denotes 0, 1, 2, 3 or 4,

and pharmaceutically usable solvates, salts, tautomers and stereoisomers thereof, including mixtures thereof in all ratios.

**13.** Compounds according to Claim 1, selected from the group

| Compound No. | Name and/or structure |
|---|---|
| "A1" | 4-Butyl-6-(1H-pyrrolo[2,3-b]pyridin-3-yl)pyrimidin-2-ylamine |
| "A2" | 4-Phenyl-6-(1H-pyrrolo[2,3-b]pyridin-3-yl)pyrimidin-2-ylamine |
| "A3" | 4-Propyl-6-(1H-pyrrolo[2,3-b]pyridin-3-yl)pyrimidin-2-ylamine |
| "A4" | 4-Methoxymethyl-6-(1H-pyrrolo[2,3-b]pyridin-3-yl)-pyrimidin-2-ylamine 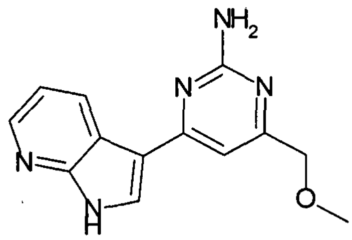 |
| "A5" | 4-Ethyl-6-(1H-pyrrolo[2,3-b]pyridin-3-yl)pyrimidin-2-ylamine |
| "A6" | 4-Pentyl-6-(1H-pyrrolo[2,3-b]pyridin-3-yl)pyrimidin-2-ylamine |
| "A7" | 4-(1-Benzyl-1H-pyrrolo[2,3-b]pyridin-3-yl)-6-butyl-pyrimidin-2-ylamine |
| "A7a" | 4-[1-(4-Methoxybenzyl)-1H-pyrrolo[2,3-b]pyridin-3-yl]-6-methoxymethylpyrimidin-2-ylamine |
| "A8" | 4-(4-Propylcyclohexyl)-6-(1H-pyrrolo[2,3-b]pyridin-3-yl)-pyrimidin-2-ylamine 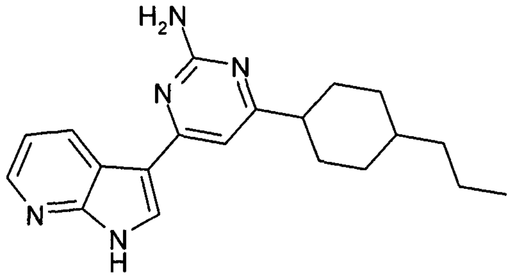 |
| "A9" | 4-(4-Propylphenyl)-6-(1H-pyrrolo[2,3-b]pyridin-3-yl)-pyrimidin-2-ylamine |
| "A10" | 4-Methyl-6-(1H-pyrrolo[2,3-b]pyridin-3-yl)pyrimidin-2-ylamine |
| "A11" | 4-Hydroxymethyl-6-(1H-pyrrolo[2,3-b]pyridin-3-yl)-pyrimidin-2-ylamine |
| "A12" | 2-[2-Amino-6-(1H-pyrrolo[2,3-b]pyridin-3-yl)pyrimidin-4-ylmethyl]isoindole-1,3-dione |
| "A13" | 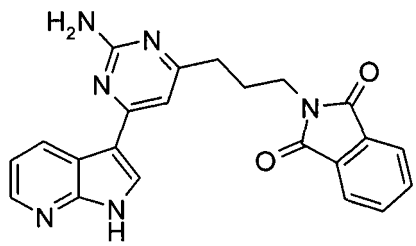 |

(continued)

| Compound No. | Name and/or structure |
|---|---|
| "A14" | 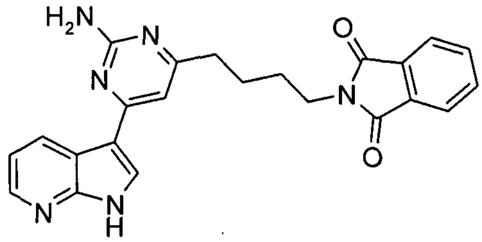 |
| "A15" | 4-Aminomethyl-6-(pyrrolo[2,3-b]pyridin-3-yl)pyrimidin-2-ylamine |
| "A16" | 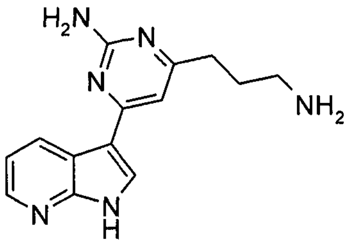 |
| "A17" | 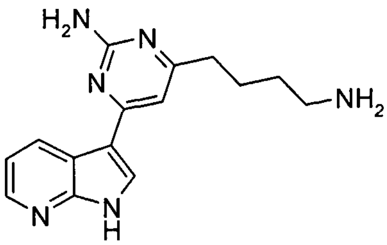 |
| "A18" | 4-(N,N-Dimethylaminomethyl)-6-(pyrrolo[2,3-b]pyridin-3-yl)pyrimidin-2-ylamine |
| "A19" | 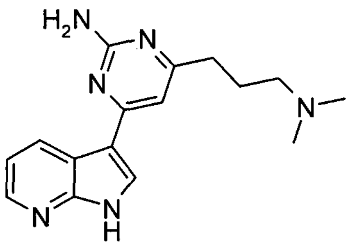 |
| "A20" | 4-(Pyrrolidin-1-ylpropyl)-6-(1H-pyrrolo[2,3-b]pyridin-3-yl)pyrimidin-2-ylamine |
| "A21" | 4-(Piperidin-4-ylmethyl)-6-(1H-pyrrolo[2,3-b]pyridin-3-yl)pyrimidin-2-ylamine |
| "A22" | 4-(1-Methylpiperidin-4-ylmethyl)-6-(1H-pyrrolo-[2,3-b]pyridin-3-yl)pyrimidin-2-ylamine |
| "A23" | 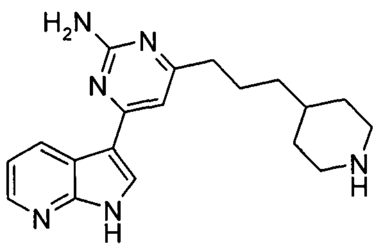 |

(continued)

| Compound No. | Name and/or structure |
|---|---|
| "A24" | 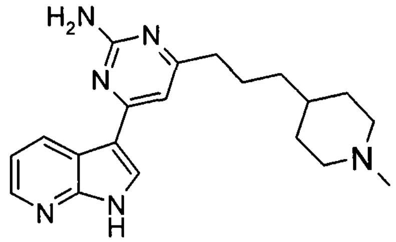 |
| "A25" | 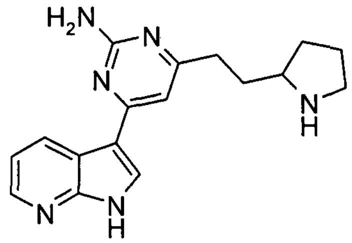 |
| "A26" | 3-[2-Amino-6-(1H-pyrrolo[2,3-b]pyridin-3-yl)pyrimidin-4-yl]propan-1-ol |
| "A27" | 4-Methoxymethyl-6-(1H-pyrrolo[2,3-b]pyridin-3-yl)-pyrimidin-2-ylamine |
| "A28" | 3-[2-Amino-6-(1H-pyrrolo[2,3-b]pyridin-3-yl)pyrimidin-4-yl]propionic acid |
| "A29" | 4-[2-Amino-6-(1H-pyrrolo[2,3-b]pyridin-3-yl)pyrimidin-4-yl]butan-1-ol |
| "A30" | 4-[2-Amino-6-(1H-pyrrolo[2,3-b]pyridin-3-yl)pyrimidin-4-yl]butyric acid |
| "A31" | 4-Methyl-6-(1H-pyrrolo[2,3-b]pyridin-3-yl)pyrimidin-2-ylamine |
| "A32" | 4-(5-Bromo-1H-pyrrolo[2,3-b]pyridin-3-yl)-6-ethyl-pyrimidin-2-ylamine |
| "A33" | 4-(3-Nitrophenyl)-6-(1H-pyrrolo[2,3-b]pyridin-3-yl)-pyrimidin-2-ylamine |
| "A34" | 4-(3-Aminophenyl)-6-(1H-pyrrolo[2,3-b]pyridin-3-yl)-pyrimidin-2-ylamine |
| "A35" | 4-Ethyl-6-(2-methyl-1H-pyrrolo[2,3-b]pyridin-3-yl)-pyrimidin-2-ylamine |
| "A36" | 4-(4-Chloro-1H-pyrrolo[2,3-b]pyridin-3-yl)-6-ethyl-pyrimidin-2-ylamine |
| "A37" | 3-(2-Amino-6-ethylpyrimidin-4-yl)-1H-pyrrolo-[2,3-b]pyridine-5-carbonitrile |
| "A38" | [2-Amino-6-(1-benzyl-1H-pyrrolo[2,3-b]pyridin-3-yl)-pyrimidin-4-yl]methanol |
| "A39" | 4-(3-Piperazin-1-ylpropyl)-6-(1H-pyrrolo[2,3-b]pyridin-3-yl)pyrimidin-2-ylamine |
| "A40" | 4-(3-Morpholin-4-ylpropyl)-6-(1H-pyrrolo[2,3-b]pyridin-3-yl)pyrimidin-2-ylamine |
| "A41" | 4-[3-(4-Methylpiperazin-1-yl)propyl]-6-(1H-pyrrolo-[2,3-b]pyridin-3-yl)pyrimidin-2-ylamine |
| "A42" | 4-Ethyl-6-{5-[1-(1-methylpiperidin-4-yl)-1H-pyrazol-4-yl]-1H-pyrrolo[2,3-b]pyridin-3-yl}pyrimidin-2-ylamine |
| "A43" | 4-(3-Aminopropyl)-6-(5-furan-2-yl-1H-pyrrolo-[2,3-b]pyridin-3-yl)pyrimidin-2-ylamine |
| "A44" | 4-(3-Aminopropyl)-6-[5-(1-methyl-1H-pyrazol-4-yl)-1H-pyrrolo[2,3-b]pyridin-3-yl]pyrimidin-2-ylamine |
| "A45" | 4-Ethyl-6-[5-(1-methyl-1H-pyrazol-4-yl)-1H-pyrrolo-[2,3-b]pyridin-3-yl]pyrimidin-2-ylamine |
| "A46" | 4-Ethyl-6-(5-pyridin-3-yl-1H-pyrrolo[2,3-b]pyridin-3-yl)-pyrimidin-2-ylamine |
| "A47" | 4-Ethyl-6-(5-pyridin-4-yl-1H-pyrrolo[2,3-b]pyridin-3-yl)-pyrimidin-2-ylamine |
| "A48" | 4-[5-(6-Aminopyridin-3-yl)-1H-pyrrolo[2,3-b]pyridin-3-yl]-6-ethylpyrimidin-2-ylamine |
| "A49" | 4-Ethyl-6-(5-furan-2-yl-1H-pyrrolo[2,3-b]pyridin-3-yl)-pyrimidin-2-ylamine |
| "A50" | 3-Amino-5-[3-(2-amino-6-ethylpyrimidin-4-yl)-1H-pyrrolo[2,3-b]pyridin-5-yl]benzoic acid |

(continued)

| Compound No. | Name and/or structure |
|---|---|
| "A51" | 4-[5-(3,5-Dimethylisoxazol-4-yl)-1H-pyrrolo-[2,3-b]pyridin-3-yl]-6-ethylpyrimidin-2-ylamine |
| "A52" | 4-Ethyl-6-(5-phenyl-1H-pyrrolo[2,3-b]pyridin-3-yl)-pyrimidin-2-ylamine |
| "A53" | 4-[5-(6-Dimethylaminopyridin-3-yl)-1H-pyrrolo-[2,3-b]pyridin-3-yl]-6-ethylpyrimidin-2-ylamine |
| "A54" | 3-[3-(2-Amino-6-ethylpyrimidin-4-yl)-1H-pyrrolo-[2,3-b]pyridin-5-yl]benzonitrile |
| "A55" | 4-Ethyl-6-{5-[1-(3-methoxypropyl)-1H-pyrazol-4-yl]-1H-pyrrolo[2,3-b]pyridin-3-yl}pyrimidin-2-ylamine |
| "A56" | 4-Ethyl-6-{5-[1-(2-methoxyethyl)-1H-pyrazol-4-yl]-1H-pyrrolo[2,3-b]pyridin-3-yl}pyrimidin-2-ylamine |
| "A57" | 4-Ethyl-6-[5-(6-fluoro-4-methylpyridin-3-yl)-1H-pyrrolo-[2,3-b]pyridin-3-yl]pyrimidin-2-ylamine |
| "A58" | N-Methyl-5-[3-(2-amino-6-ethylpyrimidin-4-yl)-1H-pyrrolo[2,3-b]pyridin-5-yl]pyridine-2-carboxamide |
| "A59" | 4-Ethyl-6-{5-[1-(3-methylbutyl)-1H-pyrazol-4-yl]-1H-pyrrolo[2,3-b]pyridin-3-yl}pyrimidin-2-ylamine |
| "A60" | 4-Ethyl-6-[5-(1H-pyrazol-4-yl)-1H-pyrrolo[2,3-b]pyridin-3-yl]pyrimidin-2-ylamine |
| "A61" | 4-Ethyl-6-[5-(2H-pyrazol-3-yl)-1H-pyrrolo[2,3-b]pyridin-3-yl]pyrimidin-2-ylamine |
| "A62" | 4-Ethyl-6-[5-(2-methyl-2H-pyrazol-3-yl)-1H-pyrrolo-[2,3-b]pyridin-3-yl]pyrimidin-2-ylamine |
| "A63" | 4-Ethyl-6-[5-(1-isopropyl-1H-pyrazol-4-yl)-1H-pyrrolo-[2,3-b]pyridin-3-yl]pyrimidin-2-ylamine |
| "A64" | 4-Ethyl-6-[5-(1,3,5-trimethyl-1H-pyrazol-4-yl)-1H-pyrrolo[2,3-b]pyridin-3-yl]pyrimidin-2-ylamine |
| "A65" | 4-[3-(2-Amino-6-ethylpyrimidin-4-yl)-1H-pyrrolo-[2,3-b]pyridin-5-yl]benzamide |
| "A66" | 4-Ethyl-6-[5-(1-isobutyl-1H-pyrazol-4-yl)-1H-pyrrolo-[2,3-b]pyridin-3-yl]pyrimidin-2-ylamine |
| "A67" | 4-(5-Bromo-1H-pyrrolo[2,3-b]pyridin-3-yl)-6-phenyl-pyrimidin-2-ylamine |

and pharmaceutically usable solvates, salts, tautomers and stereoisomers thereof, including mixtures thereof in all ratios.

**14.** Process for the preparation of compounds of the formula I according to Claims 1-13 and pharmaceutically usable salts, solvates, tautomers and stereoisomers thereof, **characterised in that**

a) a compound of the formula II

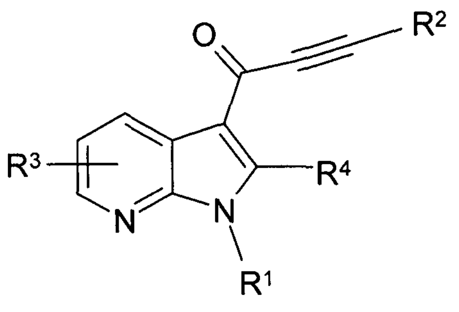

II

,

in which $R^1$ denotes an indole-protecting group, $R^2$, $R^3$ and $R^4$ have the meanings indicated in Claim 1, is reacted with guanidine,
and the indole-protecting group is simultaneously or subsequently cleaved off,
or
b) a radical $R^2$ in a compound of the formula I is converted into another $R^2$
by

i) cleaving off an amino-protecting group,
ii) cleaving an imide to give an amine,
iii) carrying out an alkylation,

and/or
a base or acid of the formula I is converted into one of its salts.

**15.** Medicaments comprising at least one compound of the formula I according to Claims 1-13 and/or pharmaceutically usable salts, solvates, tautomers and stereoisomers thereof, including mixtures thereof in all ratios, and optionally excipients and/or adjuvants.

**16.** Use of compounds according to Claims 1-13 and pharmaceutically usable salts, solvates, tautomers and stereoisomers thereof, including mixtures thereof in all ratios, for the preparation of a medicament for the treatment of tumours, tumour growth, tumour metastases and/or AIDS.

**17.** Use according to Claim 16, where the tumour originates from the group of tumours of the squamous epithelium, the bladder, the stomach, the kidneys, of head and neck, the oesophagus, the cervix, the thyroid, the intestine, the liver, the brain, the prostate, the urogenital tract, the lymphatic system, the stomach, the larynx and/or the lung.

**18.** Use according to Claim 16, where the tumour originates from the group monocytic leukaemia, lung adenocarcinoma, small-cell lung carcinomas, pancreatic cancer, colon carcinoma, glioblastomas and/or breast carcinoma.

**19.** Use according to Claim 16, where the tumour is a tumour of the blood and immune system.

**20.** Use according to Claim 16, where the tumour originates from the group of acute myeloid leukaemia, chronic myeloid leukaemia, acute lymphatic leukaemia and/or chronic lymphatic leukaemia.

**21.** Use of compounds of the formula I according to Claims 1-13 and/or physiologically acceptable salts and solvates thereof for the preparation of a medicament for the treatment of tumours, where a therapeutically effective amount of a compound of the formula I is administered in combination with a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

**22.** Use of compounds of the formula I according to Claims 1-13 and/or physiologically acceptable salts and solvates thereof for the preparation of a medicament for the treatment of tumours, where a therapeutically effective amount of a compound of the formula I is administered in combination with radiotherapy and a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

## Revendications

**1.** Composés de formule I

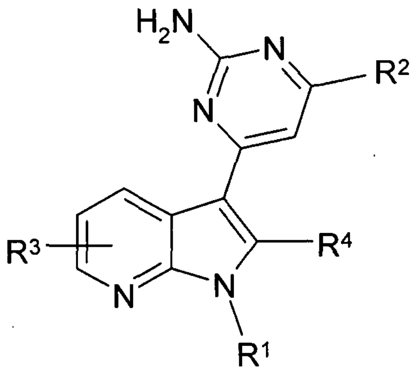

I

dans lesquels

$R^1$ désigne H, A ou $-[C(R^5)_2]_nAr$,
$R^2$ désigne A, $-[C(R^5)_2]_nAr$, $-[C(R^5)_2]_n$Hét, $-[C(R^5)_2]_n$cycloalkyle, $-[C(R^5)_2]_nOR^5$, $-(C(R^5)_2]_nN(R^5)_2$ ou $-[C(R^5)_2]_nCOOR^5$,
$R^3$ désigne H, A, Hal, CN, $-[C(R^5)_2]_nAr$, $-[C(R^5)_2]_n$Hét ou $-[C(R^5)_2]_n$cycloalkyle ou $-[C(R^5)_2]_nN(R^5)_2$,
$R^4$ désigne H, A, $-[C(R^5)_2]_nAr$, $-[C(R^5)_2]_n$Hét ou $-[C(R^5)_2]_n$cycloalkyle,
$R^5$ désigne H ou alkyle ayant 1-6 atomes de C,
A désigne alkyle non ramifié ou ramifié ayant 1-10 atomes de C, où un ou deux groupements $CH_2$ peuvent être remplacés par des atomes de O ou S et/ou par des groupements -CH=CH- et/ou de plus 1-7 atomes de H peuvent être remplacés par F,
cycloalkyle désigne alkyle cyclique ayant 3-7 atomes de C, pouvant en outre être substitué par alkyle ayant 1-6 atomes de C,
Hal désigne F, Cl, Br ou I,
Ar désigne phényle qui est non substitué ou mono-, di- ou trisubstitué par Hal, A, $OR^5$, $N(R^5)_2$, $NO_2$, CN, $COOR^5$, $CON(R^5)_2$, $NR^5COA$, $NR^5SO_2A$, $COR^5$, $SO_2N(R^5)_2$ et/ou $S(O)_pA$,
Hét désigne un hétérocycle mono- ou bicyclique saturé, insaturé ou aromatique ayant de 1 à 4 atomes de N, et/ou O et/ou S, qui est non substitué, mono-, di- ou trisubstitué par Hal, A, $OR^5$, $N(R^5)_2$, $NO_2$, CN, $COOR^5$, $CON(R^5)_2$, $NR^5COA$, $NR^5SO_2A$, $COR^5$, $SO_2NR^5$, $S(O)_pA$ et/ou =O (oxygène du carbonyle),
n désigne 0, 1, 2, 3 ou 4,
p désigne 0, 1 ou 2,

et les solvats, sels, tautomères et stéréoisomères pharmaceutiquement utilisables de ceux-ci, y compris des mélanges de ceux-ci en toutes proportions.

**2.** Composés selon la revendication 1, dans lesquels

$R^1$ désigne H ou $-[C(R^5)_2]_nAr$,

et les solvats, sels, tautomères et stéréoisomères pharmaceutiquement utilisables de ceux-ci, y compris des mélanges de ceux-ci en toutes proportions.

**3.** Composés selon la revendication 1 ou 2, dans lesquels

$R^3$ désigne H, Br, Cl, CN, Ar ou Hét,

et les solvats, sels, tautomères et stéréoisomères pharmaceutiquement utilisables de ceux-ci, y compris des mélanges de ceux-ci en toutes proportions.

**4.** Composés selon la revendication 1, 2 ou 3, dans lesquels

$R^4$ désigne H ou A,

et les solvats, sels, tautomères et stéréoisomères pharmaceutiquement utilisables de ceux-ci, y compris des mélanges de ceux-ci en toutes proportions.

**5.** Composés selon l'une ou plusieurs parmi les revendications 1-4, dans lesquels

$R^5$ désigne H ou A,

et les solvats, sels, tautomères et stéréoisomères pharmaceutiquement utilisables de ceux-ci, y compris des mélanges de ceux-ci en toutes proportions.

**6.** Composés selon l'une ou plusieurs parmi les revendications 1-5, dans lesquels

A désigne alkyle non ramifié ou ramifié ayant 1-8 atomes de C, où un ou deux groupements $CH_2$ peuvent être remplacés par oxygène et/ou de plus 1-7 atomes de H peuvent être remplacés par F,

et les solvats, sels, tautomères et stéréoisomères pharmaceutiquement utilisables de ceux-ci, y compris des mélanges de ceux-ci en toutes proportions.

**7.** Composés selon l'une ou plusieurs parmi les revendications 1-6, dans lesquels

A désigne alkyle non ramifié ou ramifié ayant 1-6 atomes de C, où un ou deux groupements $CH_2$ peuvent être remplacés par oxygène et/ou de plus 1-7 atomes de H peuvent être remplacés par F,

et les solvats, sels, tautomères et stéréoisomères pharmaceutiquement utilisables de ceux-ci, y compris des mélanges de ceux-ci en toutes proportions.

**8.** Composés selon l'une ou plusieurs parmi les revendications 1-7, dans lesquels

Ar désigne phényle qui est non substitué ou mono-, di- ou trisubstitué par A, $NO_2$, $N(R^5)_2$, CN et/ou $COOR^5$,

et les solvats, sels, tautomères et stéréoisomères pharmaceutiquement utilisables de ceux-ci, y compris des mélanges de ceux-ci en toutes proportions.

**9.** Composés selon l'une ou plusieurs parmi les revendications 1-8, dans lesquels

Hét désigne un hétérocycle mono- ou bicyclique saturé, insaturé ou aromatique ayant de 1 à 3 atomes de N et/ou O qui est non substitué ou mono-, di- ou trisubstitué par A, OH, OA, Hal, $CON(R^5)_2$, $N(R^5)_2$ et/ou =O (oxygène du carbonyle),

et les solvats, sels, tautomères et stéréoisomères pharmaceutiquement utilisables de ceux-ci, y compris des mélanges de ceux-ci en toutes proportions.

**10.** Composés selon l'une ou plusieurs parmi les revendications 1-9, dans lesquels

Hét désigne dihydropyrrolyle, pyrrolidinyle, tétrahydro-imidazolyle, dihydropyrazolyle, tétrahydropyrazolyle, dihydropyridyle, tétrahydropyridyle, pipéridinyle, morpholinyle, hexahydropyridazinyle, hexahydropyrimidinyle, pipérazinyle, 1,2,3,4-tétrahydroquinolyle, 1,2,3,4-tétrahydroisoquinolyle, 3,4-dihydrobenzo-1,4-oxazinyle, 2,3-dihydroisoindolyle, 2,3-dihydroindolyle, oxazolidinyle, isoxazolidinyle, oxadiazolidinyle, thiazolidinyle, furyle, thiényle, pyrrolyle, imidazolyle, pyrazolyle, oxazolyle, isoxazolyle, thiazolyle, pyridyle, pyrimidinyle, triazolyle, tétrazolyle, thiadiazole, pyridazinyle, pyrazinyle, indolyle, isoindolyle, benzimidazolyle, indazolyle, quinolyle ou 1,3-benzodioxolyle, chacun d'entre eux étant non substitué ou mono-, di- ou trisubstitué par A, OH, OA, Hal, $CON(R^5)_2$, $N(R^5)_2$ et/ou =O,

et les solvats, sels, tautomères et stéréoisomères pharmaceutiquement utilisables de ceux-ci, y compris des mélanges de ceux-ci en toutes proportions.

**11.** Composés selon l'une ou plusieurs parmi les revendications 1-10, dans lesquels

Hét désigne dihydropyrrolyle, pyrrolidinyle, tétrahydroimidazolyle, dihydropyrazolyle, tétrahydropyrazolyle, dihydropyridyle, tétrahydropyridyle, pipéridinyle, morpholinyle, hexahydropyridazinyle, hexahydropyrimidinyle, pipérazinyle, 1,2,3,4-tétrahydroquinolyle, 1,2,3,4-tétrahydroisoquinolyle, 3,4-dihydrobenzo-1,4-oxazinyle, 2,3-dihydroisoindolyle, 2,3-dihydroindolyle, pyrazolyle, pyridyle, furyle ou isoxazole, chacun d'entre eux étant non substitué ou mono- ou di-, ou trisubstitué par A, $CON(R^5)_2$, $N(R^5)_2$ ou F et/ou =O,

et les solvats, sels, tautomères et stéréoisomères pharmaceutiquement utilisables de ceux-ci, y compris des mélanges de ceux-ci en toutes proportions.

**12.** Composés selon l'une ou plusieurs parmi les revendications 1-11, dans lesquels

$R^1$ désigne H ou $-[C(R^5)_2]_nAr$,
$R^2$ désigne A, $-[C(R^5)_2]_nAr$, $-[C(R^5)_2]_n$Hét, $-[C(R^5)_2]_n$cycloalkyle, $-(C(R^5)_2]_nOR^5$, $-[C(R^5)_2]_nN(R^5)_2$ ou $-[C(R^5)_2]_nCOOR^5$,
$R^3$ désigne H, Br, Cl, CN, Ar ou Hét,

$R^4$ désigne H ou A,
$R^5$ désigne H ou A,
A désigne alkyle non ramifié ou ramifié ayant 1-6 atomes de C, où un ou deux groupements $CH_2$ peuvent être remplacés par oxygène et/ou de plus 1-7 atomes de H peuvent être remplacés par F,
Ar désigne phényle qui est non substitué ou mono-, di- ou trisubstitué par A, $NO_2$, $N(R^5)_2$, CN et/ou $COOR^5$,
Hét désigne dihydropyrrolyle, pyrrolidinyle, tétrahydroimidazolyle, dihydropyrazolyle, tétrahydropyrazolyle, dihydropyridyle, tétrahydropyridyle, pipéridinyle, morpholinyle, hexahydropyridazinyle, hexahydropyrimidinyle, pipérazinyle, 1,2,3,4-tétrahydroquinolyle, 1,2,3,4-tétrahydroisoquinolyle, 3,4-dihydrobenzo-1,4-oxazinyle, 2,3-dihydroisoindolyle, 2,3-dihydroindolyle, pyrazolyle, pyridyle, furyle ou isoxazole, chacun d'entre eux étant non substitué ou mono-, di- ou trisubstitué par A, $CON(R^5)_2$, $N(R^5)_2$ ou F et/ou =O
n désigne 0, 1, 2, 3 ou 4,

et les solvats, sels, tautomères et stéréoisomères pharmaceutiquement utilisables de ceux-ci, y compris des mélanges de ceux-ci en toutes proportions.

**13.** Composés selon la revendication 1, choisis dans le groupe constitué par

| Composé n° | Nom et/ou structure |
|---|---|
| "A1" | 4-Butyl-6-(1H-pyrrolo[2,3-b]pyridin-3-yl)pyrimidin-2-ylamine |
| "A2" | 4-Phényl-6-(1H-pyrrolo[2,3-b]pyridin-3-yl)pyrimidin-2-ylamine |
| "A3" | 4-Propyl-6-(1H-pyrrolo[2,3-b]pyridin-3-yl)pyrimidin-2-ylamine |
| "A4" | 4-Méthoxyméthyl-6-(1H-pyrrolo[2,3-b]pyridin-3-yl)-pyrimidin-2-ylamine 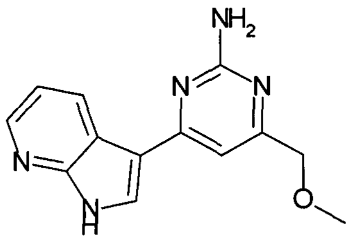 |
| "A5" | 4-Éthyl-6-(1H-pyrrolo[2,3-b]pyridin-3-yl)pyrimidin-2-ylamine |
| "A6" | 4-Pentyl-6-(1H-pyrrolo[2,3-b]pyridin-3-yl)pyrimidin-2-ylamine |
| "A7" | 4-(1-Benzyl-1H-pyrrolo[2,3-b]pyridin-3-yl)-6-butylpyrimidin-2-ylamine |
| "A7a" | 4-[1-(4-Méthoxybenzyl)-1H-pyrrolo[2,3-b]pyridin-3-yl]-6-méthoxyméthylpyrimidin-2-ylamine |
| "A8" | 4-(4-Propylcyclohexyl)-6-(1H-pyrrolo[2,3-b]pyridin-3-yl)-pyrimidin-2-ylamine 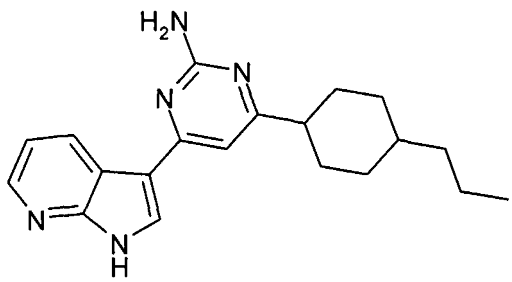 |
| "A9" | 4-(4-Propylphényl)-6-(1H-pyrrolo[2,3-b]pyridin-3-yl)-pyrimidin-2-ylamine |
| "A10" | 4-Méthyl-6-(1H-pyrrolo[2,3-b]pyridin-3-yl)pyrimidin-2-ylamine |
| "A11" | 4-Hydroxyméthyl-6-(1H-pyrrolo[2,3-b]pyridin-3-yl)-pyrimidin-2-ylamine |
| "A12" | 2-[2-Amino-6-(1H-pyrrolo[2,3-b]pyridin-3-yl)pyrimidin-4-ylméthyl]isoindole-1,3-dione |

(suite)

| Composé n° | Nom et/ou structure |
|---|---|
| "A13" | 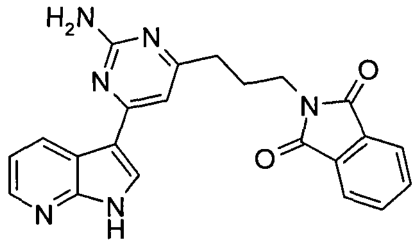 |
| "A14" | 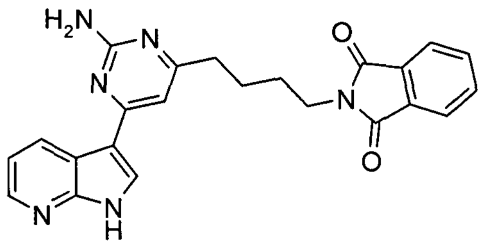 |
| "A15" | 4-Aminométhyl-6-(pyrrolo[2,3-b]pyridin-3-yl)pyrimidin-2-ylamine |
| "A16" | 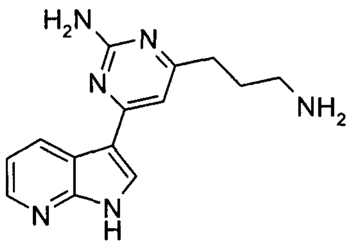 |
| "A17" | 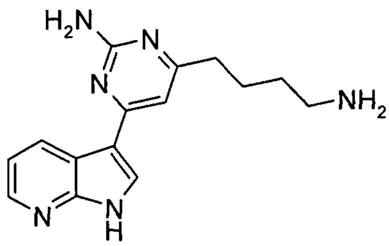 |
| "A18" | 4-(N,N-Diméthylaminométhyl)-6-(pyrrolo[2,3-b]pyridin-3-yl)pyrimidin-2-ylamine |
| "A19" | 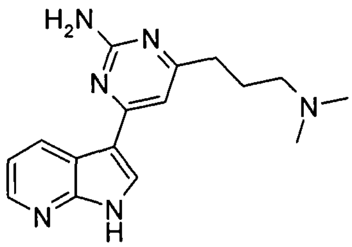 |
| "A20" | 4-(Pyrrolidin-1-ylpropyl)-6-(1H-pyrrolo[2,3-b]pyridin-3-yl)pyrimidin-2-ylamine |
| "A21" | 4-(Pipéridin-4-ylméthyl)-6-(1H-pyrrolo[2,3-b]pyridin-3-yl)pyrimidin-2-ylamine |
| "A22" | 4-(1-Méthylpipéridin-4-ylméthyl)-6-(1H-pyrrolo[2,3-b]pyridin-3-yl)pyrimidin-2-ylamine |

(suite)

| Composé n° | Nom et/ou structure |
|---|---|
| "A23" | 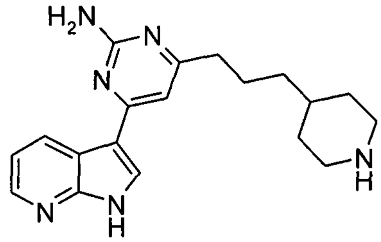 |
| "A24" | 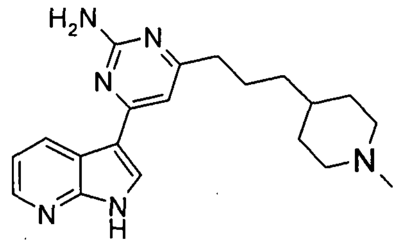 |
| "A25" | 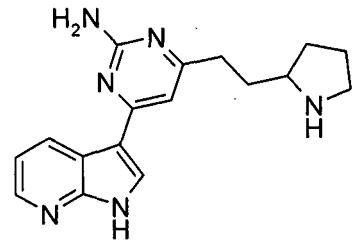 |
| "A26" | 3-[2-Amino-6-(1H-pyrrolo[2,3-b]pyridin-3-yl)pyrimidin-4-yl]propan-1-ol |
| "A27" | 4-Méthoxyméthyl-6-(1H-pyrrolo[2,3-b]pyridin-3-yl)-pyrimidin-2-ylamine |
| "A28" | Acide 3-[2-amino-6-(1H-pyrrolo[2,3-b]pyridin-3-yl)-pyrimidin-4-yl]propionique |
| "A29" | 4-[2-Amino-6-(1H-pyrrolo[2,3-b]pyridin-3-yl)pyrimidin-4-yl]butan-1-ol |
| "A30" | Acide 4-[2-amino-6-(1H-pyrrofo[2,3-b]pyridin-3-yl)-pyrimidin-4-yl]butyrique |
| "A31" | 4-Méthyl-6-(1H-pyrrolo[2,3-b]pyridin-3-yl)pyrimidin-2-ylamine |
| "A32" | 4-(5-Bromo-1H-pyrrolo[2,3-b]pyridin-3-yl)-6-éthylpyrimidin-2-ylamine |
| "A33" | 4-(3-Nitrophényl)-6-(1H-pyrrolo[2,3-b]pyridin-3-yl)-pyrimidin-2-ylamine |
| "A34" | 4-(3-Aminophényl)-6-(1H-pyrrolo[2,3-b]pyridin-3-yl)-pyrimidin-2-ylamine |
| "A35" | 4-Éthyl-6-(2-méthyl-1H-pyrrolo[2,3-b]pyridin-3-yl)-pyrimidin-2-ylamine |
| "A36" | 4-(4-Chloro-1H-pyrrolo[2,3-b]pyridin-3-yl)-6-éthylpyrimidin-2-ylamine |
| "A37" | 3-(2-Amino-6-éthylpyrimidin-4-yl)-1H-pyrrolo-[2,3-b]pyridine-5-carbonitrile |
| "A38" | [2-Amino-6-(1-benzyl-1H-pyrrolo[2,3-b]pyridin-3-yl)-pyrimidin-4-yl]méthanol |
| "A39" | 4-(3-Pipérazin-1-ylpropyl)-6-(1H-pyrrolo[2,3-b]pyridin-3-yl)pyrimidin-2-ylamine |
| "A40" | 4-(3-Morpholin-4-ylpropyl)-6-(1H-pyrrolo[2,3-b]pyridin-3-yl)pyrimidin-2-ylamine |
| "A41" | 4-[3-(4-Méthylpipérazin-1-yl)propyl]-6-(1H-pyrrolo[2,3-b]pyridin-3-yl)pyrimidin-2-ylamine |
| "A42" | 4-Éthyl-6-{5-[1-(1-méthylpipéridin-4-yl)-1H-pyrazol-4-yl]-1H-pyrrolo[2,3-b]pyridin-3-yl}pyrimidin-2-ylamine |
| "A43" | 4-(3-Aminopropyl)-6-(5-furan-2-yl-1H-pyrrolo[2,3-b]pyridin-3-yl)pyrimidin-2-ylamine |
| "A44" | 4-(3-Aminopropyl)-6-[5-(1-méthyl-1H-pyrazol-4-yl)-1H-pyrrolo[2,3-b]pyridin-3-yl]pyrimidin-2-ylamine |

(suite)

| Composé n° | Nom et/ou structure |
|---|---|
| "A45" | 4-Éthyl-6-[5-(1-méthyl-1H-pyrazol-4-yl)-1H-pyrrolo[2,3-b]pyridin-3-yl]pyrimidin-2-ylamine |
| "A46" | 4-Éthyl-6-(5-pyridin-3-yl-1H-pyrrolo[2,3-b]pyridin-3-yl)-pyrimidin-2-ylamine |
| "A47" | 4-Éthyl-6-(5-pyridin-4-yl-1H-pyrrolo[2,3-b]pyridin-3-yl)-pyrimidin-2-ylamine |
| "A48" | 4-[5-(6-Aminopyridin-3-yl)-1H-pyrrolo[2,3-b]pyridin-3-yl]-6-éthylpyrimidin-2-ylamine |
| "A49" | 4-Éthyl-6-(5-furan-2-yl-1H-pyrrolo[2,3-b]pyridin-3-yl)-pyrimidin-2-ylamine |
| "A50" | Acide 3-amino-5-[3-(2-amino-6-éthylpyrimidin-4-yl)-1H-pyrrolo[2,3-b]pyridin-5-yl]benzoïque |
| "A51" | 4-[5-(3,5-Diméthylisoxazol-4-yl)-1H-pyrrolo[2,3-b]-pyridin-3-yl]-6-éthylpyrimidin-2-ylamine |
| "A52" | 4-Éthyl-6-(5-phényl-1H-pyrrolo[2,3-b]pyridin-3-yl)-pyrimidin-2-ylamine |
| "A53" | 4-[5-(6-Diméthylaminopyridin-3-yl)-1H-pyrrolo[2,3-b]-pyridin-3-yl]-6-éthylpyrimidin-2-ylamine |
| "A54" | 3-[3-(2-Amino-6-éthylpyrimidin-4-yl)-1H-pyrrolo[2,3-b]-pyridin-5-yl]benzonitrile |
| "A55" | 4-Éthyl-6-{5-[1-(3-méthoxypropyl)-1H-pyrazol-4-yl]-1H-pyrrolo[2,3-b]pyridin-3-yl}pyrimidin-2-ylamine |
| "A56" | 4-Éthyl-6-{5-[1-(2-méthoxyéthyl)-1H-pyrazol-4-yl]-1H-pyrrolo[2,3-b]pyridin-3-yl}pyrimidin-2-ylamine |
| "A57" | 4-Éthyl-6-[5-(6-fluoro-4-méthylpyridin-3-yl)-1H-pyrrolo[2,3-b]pyridin-3-yl]pyrimidin-2-ylamine |
| "A58" | N-Méthyl-5-[3-(2-amino-6-éthylpyrimidin-4-yl)-1H-pyrrolo[2,3-b]pyridin-5-yl]pyridine-2-carboxamide |
| "A59" | 4-Éthyl-6-{5-[1-(3-méthylbutyl)-1H-pyrazol-4-yl]-1H-pyrrolo[2,3-b]pyridin-3-yl}pyrimidin-2-ylamine |
| "A60" | 4-Ethyl-6-[5-(1H-pyrazol-4-yl)-1H-pyrrolo[2,3-b]pyridin-3-yl]pyrimidin-2-ylamine |
| "A61" | 4-Éthyl-6-[5-(2H-pyrazol-3-yl)-1H-pyrrolo[2,3-b]pyridin-3-yl]pyrimidin-2-ylamine |
| "A62" | 4-Éthyl-6-[5-(2-méthyl-2H-pyrazol-3-yl)-1H-pyrrolo[2,3-b]pyridin-3-yl]pyrimidin-2-ylamine |
| "A63" | 4-Éthyl-6-[5-(1-isopropyl-1H-pyrazol-4-yl)-1H-pyrrolo[2,3-b]pyridin-3-yl]pyrimidin-2-ylamine |
| "A64" | 4-Éthyl-6-[5-(1,3,5-triméthyl-1H-pyrazol-4-yl)-1H-pyrrolo[2,3-b]pyridin-3-yl]pyrimidin-2-ylamine |
| "A65" | 4-[3-(2-Amino-6-éthylpyrimidin-4-yl)-1H-pyrrolo[2,3-b]pyridin-5-yl]benzamide |
| "A66" | 4-Éthyl-6-[5-(1-isobutyl-1H-pyrazol-4-yl)-1H-pyrrolo[2,3-b]pyridin-3-yl]pyrimidin-2-ylamine |
| "A67" | 4-(5-Bromo-1H-pyrrolo[2,3-b]pyridin-3-yl)-6-phénylpyrimidin-2-ylamine |

et les solvats, sels, tautomères et stéréoisomères pharmaceutiquement utilisables de ceux-ci, y compris des mélanges de ceux-ci en toutes proportions.

**14.** Procédé de préparation de composés de formule I selon les revendications 1-13 et de sels, solvats, tautomères et stéréoisomères pharmaceutiquement utilisables de ceux-ci, **caractérisé en ce que**

a) un composé de formule II

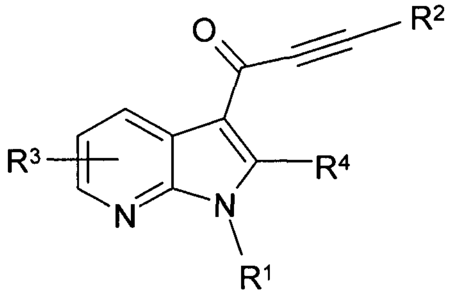

II

,

dans laquelle $R^1$ désigne un groupement protecteur d'indole,
$R^2$, $R^3$ et $R^4$ revêtent les significations indiquées selon la revendication 1,
est réagi avec de la guanidine,
et le groupement protecteur d'indole est éliminé par clivage simultanément ou par la suite,
ou
b) un radical $R^2$ dans un composé de formule I est converti en un autre $R^2$
par

i) clivage d'un groupement protecteur d'amino,
ii) clivage d'un imide pour donner une amine,
iii) réalisation d'une alkylation,

et/ou
une base ou un acide de formule I est converti(e) en l'un de ses sels.

**15.** Médicaments comprenant au moins un composé de formule I selon les revendications 1-13 et/ou des sels, solvats, tautomères et stéréoisomères pharmaceutiquement utilisables de celui-ci, y compris des mélanges de ceux-ci en toutes proportions, et éventuellement des excipients et/ou des adjuvants.

**16.** Utilisation de composés selon les revendications 1-13 et de sels, solvats, tautomères et stéréoisomères pharmaceutiquement utilisables de ceux-ci, y compris des mélanges de ceux-ci en toutes proportions, pour la préparation d'un médicament destiné au traitement de tumeurs, de la croissance tumorale, de métastases tumorales et/ou du SIDA.

**17.** Utilisation selon la revendication 16, dans laquelle la tumeur provient du groupe constitué par les tumeurs de l'épithélium squameux, de la vessie, de l'estomac, du rein, de la tête et du cou, de l'oesophage, du col de l'utérus, de la thyroïde, des intestins, du foie, du cerveau, de la prostate, du tractus urogénital, du système lymphatique, de l'estomac, du larynx et/ou du poumon.

**18.** Utilisation selon la revendication 16, dans laquelle la tumeur provient du groupe constitué par la leucémie monocytique, l'adénocarcinome du poumon, les carcinomes du poumon à petites cellules, le cancer du pancréas, le carcinome du côlon, les glioblastomes et/ou le carcinome du sein.

**19.** Utilisation selon la revendication 16, dans laquelle la tumeur est une tumeur du sang et du système immunitaire.

**20.** Utilisation selon la revendication 16, dans laquelle la tumeur provient du groupe constitué par la leucémie myéloïde aiguë, la leucémie myéloïde chronique, la leucémie lymphatique aiguë et/ou la leucémie lymphatique chronique.

**21.** Utilisation de composés de formule I selon les revendications 1-13 et/ou de sels et de solvats physiologiquement acceptables de ceux-ci, pour la préparation d'un médicament destiné au traitement de tumeurs, dans laquelle une quantité thérapeutiquement efficace d'un composé de formule I est administrée en association avec un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.

**22.** Utilisation de composés de formule I selon les revendications 1-13 et/ou de sels et de solvats physiologiquement acceptables de ceux-ci, pour la préparation d'un médicament destiné au traitement de tumeurs, dans laquelle une quantité thérapeutiquement efficace d'un composé de formule I est administrée en association avec une radiothérapie et un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2004089913 A
- WO 2005095400 A
- WO 2006124863 A
- WO 0050032 A
- US 6069134 A
- WO 0059503 A


### In der Beschreibung aufgeführte Nicht-Patentliteratur


- **P.M. FRESNEDA et al.** *Tetrahedron,* 2001, vol. 57, 2355-2363
- *Bioorganic & Medicinal Chemistry Letters,* 2006, vol. 16 (18), 4818-4821
- *Int. J. Pharm.,* 1995, vol. 115, 61-67
- *Pharmaceutical Research,* 1986, vol. 3 (6), 318